# EUROPEAN PATENT APPLICATION

(11) **EP 4 253 545 A1**
(43) Date of publication of application: **04.10.2023**
(21) Application number: 21897474.9
(22) Date of filing: 22.09.2021
(51) Int. Cl.: C12N 15/115

(54) **SARS-COV-2 SPIKE GLYCOPROTEIN-BINDING NUCLEIC ACID MOLECULE, SARS-COV-2 DETECTION SENSOR, SARS-COV-2 DETECTION REAGENT, SARS-COV-2 DETECTION METHOD, AND SARS-COV-2 VIRUS DEACTIVATOR**

(30) Priority: 30.11.2020 JP 2020198556
(71) Applicant: NEC Solution Innovators, Ltd., Koto-ku, Tokyo 136-8627 (JP)
(72) Inventor: MINAGAWA Hirotaka, Tokyo 136-8627 (JP); FUJITA Tomoko, Tokyo 136-8627 (JP); KATO Shintaro, Tokyo 136-8627 (JP); INAGUMA Asumi, Tokyo 136-8627 (JP); HORII Katsunori, Tokyo 136-8627 (JP)
(74) Representative: Betten & Resch
(86) International application number: PCT/JP2021/034667
(87) International publication number: WO 2022/113496

(57) **Abstract**

An example object of the invention is to provide a binding nucleic acid molecule capable of specifically binding to a SARS-CoV-2 spike glycoprotein. A SARS-CoV-2 spike glycoprotein-binding nucleic acid molecule according to an example aspect of the invention includes: any of the following polynucleotides (a), and (b):
(a) a polynucleotide consisting of any of base sequences of SEQ ID NOs: 1 to 7 or a partial sequence of any of the base sequences of SEQ ID NOs: 1 to 7;
(b) a polynucleotide consisting of a base sequence having 80% or more identity to any base sequence of the polynucleotide (a), and binding to a SARS-CoV-2 spike glycoprotein:

## Description

The present invention relates to a SARS-CoV-2 spike glycoprotein-binding nucleic acid molecule, a SARS-CoV-2 detection sensor, a SARS-CoV-2 detection reagent, a SARS-CoV-2 detection method, and a SARS-CoV-2 virus deactivator.

### BACKGROUND ART

Recently, the global pandemic of SARS-CoV-2 (also called "2019-nCoV"), a new type of coronavirus, has become a problem, and elucidation of the mechanism of infection by SARS-CoV-2 and therapeutic methods and vaccines are being developed.

Hence, in order to detect SARS-CoV-2, an attempt has been made to obtain aptamers targeting proteins constituting SARS-CoV-2 (Non-Patent Literature 1).

### Citation List

### Non Patent Literature

Non Patent Literature 1: Song Y et al, Anal. Chem. (2020), 92, 9895-9900

### SUMMARY

### Technical Problem

There is a need for aptamers capable of binding specifically to SARS-CoV-2, having high binding ability and slow dissociation.

An example object of the invention is to provide a binding nucleic acid molecule capable of specifically binding to a SARS-CoV-2 spike glycoprotein involved in binding to angiotensin-converting enzyme 2 (ACE2), which is a target of SARS-CoV-2, and having high binding ability and slow dissociation.

### Solution to Problem

A SARS-CoV-2 spike glycoprotein-binding nucleic acid molecule (hereinafter, also referred to as "nucleic acid") according to an example aspect of the invention includes:
any of the following polynucleotides (a), (b), (c) and (d):
(a) a polynucleotide consisting of any of base sequences of SEQ ID NOs: 1 to 7 or a partial sequence of any of the base sequences of SEQ ID NOs: 1 to 7;
(b) a polynucleotide consisting of a base sequence having 80% or more identity to any base sequence of the polynucleotide (a), and binding to a SARS-CoV-2 spike glycoprotein;
(c) a polynucleotide consisting of a base sequence complementary to a polynucleotide that hybridizes to a polynucleotide consisting of any base sequence of the polynucleotide (a) under a stringent condition, and binding to a SARS-CoV-2 spike glycoprotein; and (d) a polynucleotide consisting of a base sequence obtained by deletion, substitution, insertion, and/or addition of one to several bases in any base sequence of the polynucleotide (a), and binding to a SARS-CoV-2 spike glycoprotein:
   SEQ ID NO: 1: 
GGTATGTCTCCGCCACTGAAATCCGTGCCTAATCTCACCCCACGGAATTCATGGCA
AAGCCGAGGTGTCTTGTATTC;
   SEQ ID NO: 2: 
GGTATGTCTCCGCCACTGAAATCTAATCTCACATTGTAAGCAAAGGAGAATAAGCA
AAGCCGAGGTGTCTTGTATTC;
   SEQ ID NO: 3: 
GGTATGTCTCCGCCACTGAAATCCCTGACCGCTGACCAAATCTCAGTGCAGATGCA
AAGCCGAGGTGTCTTGTATTC;
   SEQ ID NO: 4: 
GGTATGTCTCCGCCACTGAAATCTTGTCCCCTAATAGGTTCCGACTGACAAGTGCA
AAGCCGAGGTGTCTTGTATTC;
   SEQ ID NO: 5: 
GGTTTAGCCCTCGACTCCAAATGTGCACTCTCCCGGTATCCCTAATCTCACCCGATA
CCGTTGATGTGCTGCCAATAC;
   SEQ ID NO: 6: 
GGTTTAGCCCTCGACTCCAAATGACATGAGCCAGGTGCATCTTGAACGTCATAGAT
ACCGTTGATGTGCTGCCAATAC;
   SEQ ID NO: 7:

GGTTTAGCCCTCGACTCCAAATGTGCTGATACTCGGTATCCCTAATCTCACCCGATA
CCGTTGATGTGCTGCCAATAC.

A SARS-CoV-2 detection sensor according to an example aspect of the invention includes:
the SARS-CoV-2 spike glycoprotein-binding nucleic acid molecule according to an example aspect of the invention.

A SARS-CoV-2 detection reagent according to an example aspect of the invention includes:
the SARS-CoV-2 spike glycoprotein-binding nucleic acid molecule according to an example aspect of the invention.

A method for detecting SARS-CoV-2 according to an example aspect of the invention includes:
detecting a SARS-CoV-2 spike glycoprotein in the sample by bringing a sample into contact with a nucleic acid molecule; wherein
the nucleic acid molecule is the SARS-CoV-2 spike glycoprotein-binding nucleic acid molecule according to an example aspect of the invention, and
in the detecting, the SARS-CoV-2 spike glycoprotein in the sample is bonded to the nucleic acid molecule to detect SARS-CoV-2 in the sample.

### Advantageous Effects of Invention

A SARS-CoV-2 spike glycoprotein-binding nucleic acid molecule according to an example aspect of the invention is capable of specifically binding to a SARS-CoV-2 spike glycoprotein, and having high binding ability and slow dissociation. Therefore, according to a SARS-CoV-2 spike glycoprotein-binding nucleic acid molecule according to an example aspect of the invention, for example, SARS-CoV-2 can be detected with excellent accuracy by detecting whether or not the SARS-CoV-2 spike glycoprotein-binding nucleic acid molecule is bonded to a SARS-CoV-2 spike glycoprotein in the sample.

### BRIEF DESCRIPTION OF DRAWINGS

[FIG. 1] FIG. 1 shows graphs showing the ability of aptamers to bind to SARS-CoV-2 spike glycoproteins in Example 1.
[FIG. 2] FIG. 2 shows graphs showing the ability of aptamers to bind to SARS-CoV-2 spike glycoproteins in Example 2.
[FIG. 3] FIG. 3 shows graphs showing the ability of aptamers to bind to SARS-CoV-2 spike glycoproteins in Example 2.
[FIG. 4] FIG. 4 shows graphs showing the ability of aptamers to bind to SARS-CoV-2 spike glycoproteins in Example 2.
[FIG. 5] FIG. 5 shows graphs showing the ability of aptamers to bind to SARS-CoV-2 spike glycoproteins in Example 2.
[FIG. 6] FIG. 6 shows graphs showing the ability of aptamers to bind to SARS-CoV-2 spike glycoproteins in Example 2.
[FIG. 7] FIG. 7 is a schematic diagram showing the sequence of a SARS-CoV-2 spike glycoprotein.
[FIG. 8] FIG. 8 is a graph showing the ability of aptamers to bind to SARS-CoV-2 spike glycoproteins in Example 3.

### EXAMPLE EMBODIMENTS

### (1) SARS-CoV-2 spike glycoprotein-binding nucleic acid molecule

The SARS-CoV-2 spike glycoprotein-binding nucleic acid molecule of the present invention includes:
any of the following polynucleotides (a), (b), (c) and (d):
(a) a polynucleotide consisting of any of base sequences of SEQ ID NOs: 1 to 7 or a partial sequence of any of the base sequences of SEQ ID NOs: 1 to 7;
(b) a polynucleotide consisting of a base sequence having 80% or more identity to any base sequence of the polynucleotide (a), and binding to a SARS-CoV-2 spike glycoprotein;
(c) a polynucleotide consisting of a base sequence complementary to a polynucleotide that hybridizes to a polynucleotide consisting of any base sequence of the polynucleotide (a) under a stringent condition, and binding to a SARS-CoV-2 spike glycoprotein; and
(d) a polynucleotide consisting of a base sequence obtained by deletion, substitution, insertion, and/or addition of one to several bases in any base sequence of the polynucleotide (a), and binding to a SARS-CoV-2 spike glycoprotein.

As used herein, the "SARS-CoV-2" means severe acute respiratory syndrome coronavirus 2. Infection caused by SARS-CoV-2 is also called COVID-19. SARS-Cov-2 is a virus classified in the genus Beta Coronavirus. A coronavirus containing SARS-CoV-2 includes an envelope composed of an envelope protein, a membrane protein, a spike glycoprotein, and the like, wherein a single-stranded RNA which is a genomic RNA is covered with the envelope. In the spike glycoprotein, the periphery amino acid may or may not be modified with a sugar chain.

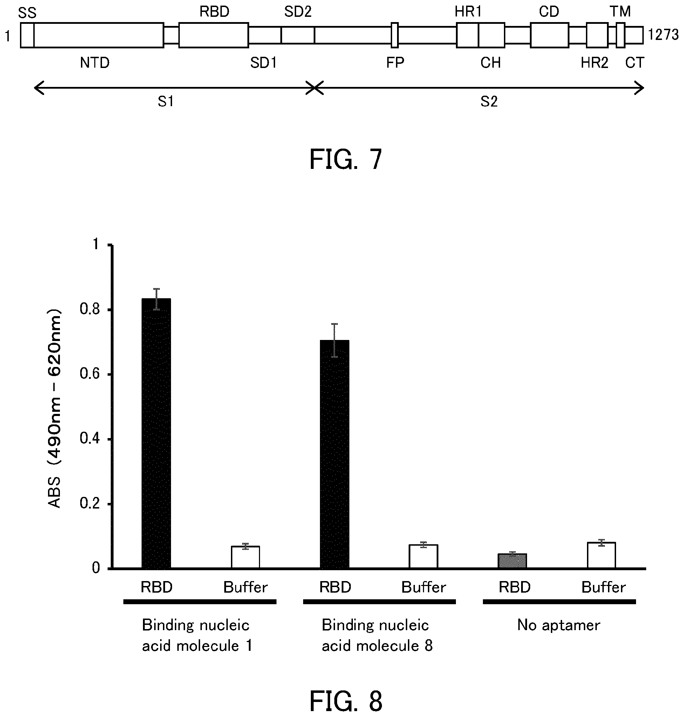

The nucleic acid molecule of the present invention is capable of binding to a SARS-CoV-2 spike glycoprotein, which is a protein constituting a SARS-CoV-2, as described above. The SARS-CoV-2 spike glycoprotein is involved in binding to angiotensin-converting enzyme 2 (ACE2), which is a target of SARS-CoV-2. A schematic diagram of the sequence of a SARS-CoV-2 spike glycoprotein is shown in FIG. 7. The nucleic acid molecule of the present invention may be bonded to at least one of a RBD domain, a S1+S2 domain, a S1 domain, and a trimer of S1+S2 domain (also referred to as S-trimer) constituting a SARS-CoV-2 spike glycoprotein. The nucleic acid molecule of the present invention may be bonded to all of the RBD(receptor binding domain), the S1+S2 domain, the S1 domain, and the trimer of S1+S2 or may be bonded to the SARS-CoV-2 spike glycoprotein at regions other than these. The ability of the nucleic acid molecule of the present invention to bind to the RBD domain, the S1+S2 domain, the S1 domain, and the trimer can be examined, for example, by using four commercially available reagents shown in Table 1 in Example 1 to be described below as targets. The nucleic acid molecule of the present invention is also referred to as an aptamer hereinafter, for example.

Regarding the information of the amino acid sequence of the SARS-CoV-2 spike glycoprotein, for example, reference can be made to the following amino acid sequence (SEQ ID NO: 15), which is registered under the accession number P0DTC2 of UniProt (http://www.uniprot.org/). The aforementioned domains of the SARS-CoV-2 spike glycoprotein are represented as follows in the amino acid sequence number of the accession number P0DTC2: RBD domain: 319-541 (SEQ ID NO: 16), S1+S2 domain: 13-1273 (SEQ ID NO: 17), and S1 domains: 13-685 (SEQ ID NO: 18). One or more mutations (e.g., deletion, substitution, insertion, and/or addition) may be introduced into the amino acid sequences of the SARS-CoV-2 spike glycoprotein and the domains thereof, for example, to the extent that the nucleic acid molecule of the present invention binds thereto.
SARS-CoV-2 spike glycoprotein (SEQ ID NO: 15)
RBD domain of SARS-CoV-2 spike glycoprotein (SEQ ID NO: 16) RVQPTESIVRFPNITNLCPFGEVFNATRFASVYAWNRKRISNCVADYSVLYNSASFSTFKC 
YGVSPTKLNDLCFTNVYADSFVIRGDEVRQIAPGQTGKIADYNYKLPDDFTGCVIAWNS
NNLDSKVGGNYNYLYRLFRKSNLKPFERDISTEIYQAGSTPCNGVEGFNCYFPLQSYGFQ
PTNGVGYQPYRVVVLSFELLHAPATVCGPKKSTNLVKNKCVNF
S1+S2 domain of SARS-CoV-2 spike glycoprotein (SEQ ID NO: 17) 
SQCVNLTTRTQLPPAYTNSFTRGVYYPDKVFRSSVLHSTQDLFLPFFSNVTWFHAIHVSG
TNGTKRFDNPVLPFNDGVYFASTEKSNIIRGWIFGTTLDSKTQSLLIVNNATNVVIKVCEF
QFCNDPFLGVYYHKNNKSWMESEFRVYSSANNCTFEYVSQPFLMDLEGKQGNFKNLRE
FVFKNIDGYFKIYSKHTPINLVRDLPQGFSALEPLVDLPIGINITRFQTLLALHRSYLTPGDS
SSGWTAGAAAYYVGYLQPRTFLLKYNENGTITDAVDCALDPLSETKCTLKSFTVEKGIY
QTSNFRVQPTESIVRFPNITNLCPFGEVFNATRFASVYAWNRKRISNCVADYSVLYNSASF
STFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQ
SYGFQPTNGVGYQPYRVVVLSFELLHAPATVCGPKKSTNLVKNKCVNFNFNGLTGTGVL
TESNKKFLPFQQFGRDIADTTDAVRDPQTLEILDITPCSFGGVSVITPGTNTSNQVAVLYQ
DVNCTEVPVAIHADQLTPTWRVYSTGSNVFQTRAGCLIGAEHVNNSYECDIPIGAGICAS
YQTQTNSPRRARSVASQSIIAYTMSLGAENSVAYSNNSIAIPTNFTISVTTEILPVSMTKTSV
DCTMYICGDSTECSNLLLQYGSFCTQLNRALTGIAVEQDKNTQEVFAQVKQIYKTPPIKD
FGGFNFSQILPDPSKPSKRSFIEDLLFNKVTLADAGFIKQYGDCLGDIAARDLICAQKFNG
LTVLPPLLTDEMIAQYTSALLAGTITSGWTFGAGAALQIPFAMQMAYRFNGIGVTQNVLY
ENQKLIANQFNSAIGKIQDSLSSTASALGKLQDVVNQNAQALNTLVKQLSSNFGAISSVL
NDILSRLDKVEAEVQIDRLITGRLQSLQTYVTQQLIRAAEIRASANLAATKMSECVLGQS
KRVDFCGKGYHLMSFPQSAPHGVVFLHVTYVPAQEKNFTTAPAICHDGKAHFPREGVFV
SNGTHWFVTQRNFYEPQIITTDNTFVSGNCDVVIGIVNNTVYDPLQPELDSFKEELDKYF
KNHTSPDVDLGDISGINASVVNIQKEIDRLNEVAKNLNESLIDLQELGKYEQYIKWPWYI
WLGFIAGLIAIVMVTIMLCCMTSCCSCLKGCCSCGSCCKFDEDDSEPVLKGVKLHYT
S1 domain of SARS-CoV-2 spike glycoprotein (SEQ ID NO: 18)

SQCVNLTTRTQLPPAYTNSFTRGVYYPDKVFRSSVLHSTQDLFLPFFSNVTWFHAIHVSG
TNGTKRFDNPVLPFNDGVYFASTEKSNIIRGWIFGTTLDSKTQSLLIVNNATNVVIKVCEF
QFCNDPFLGVYYHKNNKSWMESEFRVYSSANNCTFEYVSQPFLMDLEGKQGNFKNLRE
FVFKNIDGYFKIYSKHTPINLVRDLPQGFSALEPLVDLPIGINITRFQTLLALHRSYLTPGDS
SSGWTAGAAAYYVGYLQPRTFLLKYNENGTITDAVDCALDPLSETKCTLKSFTVEKGIY
QTSNFRVQPTESIVRFPNITNLCPFGEVFNATRFASVYAWNRKRISNCVADYSVLYNSASF
STFKCYGVSPTKLNDLCFTNVYADSFVIRGDEVRQIAPGQTGKIADYNYKLPDDFTGCVI

AWNSNNLDSKVGGNYNYLYRLFRKSNLKPFERDISTEIYQAGSTPCNGVEGFNCYFPLQ
SYGFQPTNGVGYQPYRVVVLSFELLHAPATVCGPKKSTNLVKNKCVNFNFNGLTGTGVL
TESNKKFLPFQQFGRDIADTTDAVRDPQTLEILDITPCSFGGVSVITPGTNTSNQVAVLYQ
DVNCTEVPVAIHADQLTPTWRVYSTGSNVFQTRAGCLIGAEHVNNSYECDIPIGAGICAS
YQTQTNSPRRAR

As used herein, the expression "binds to a SARS-CoV-2 spike glycoprotein" refers, for example, to "having the ability to bind to a SARS-CoV-2 spike glycoprotein" or "having the activity to bind to a SARS-CoV-2 spike glycoprotein". The binding between the nucleic acid molecule of the present invention and the SARS-CoV-2 spike glycoprotein can be determined, for example, by surface plasmon resonance (SPR) analysis. As the analysis, for example, ProteOn (trade name, BioRad) can be used. The nucleic acid molecule of the present invention can be used, for example, to detect SARS-CoV-2 because it binds to the SARS-CoV-2 spike glycoprotein.

The nucleic acid molecule of the present invention may be, for example, a molecule consisting of the polynucleotide (a) or (b), or a molecule including the polynucleotide. The nucleic acid may be composed of, for example, DNA (deoxyribonucleic acid), RNA (ribonucleic acid) or DNA and RNA. When the nucleic acid molecule of the present invention is composed of DNA, the nucleic acid molecule of the present invention can be referred to as, for example, a DNA molecule or a DNA aptamer.

The polynucleotide (a) may be, for example, a polynucleotide including the base sequences of SEQ ID NOs: 1 to 7, or a polynucleotide consisting of the base sequences of SEQ ID NOs: 1 to 7. Further, the polynucleotide (a) may be a polynucleotide including a partial sequence of the base sequences of SEQ ID NOs: 1 to 7, or may be a polynucleotide consisting of the partial sequence. The partial sequence is not particularly limited, and may be, for example, a sequence obtained by deleting a sequence of at least one of the 5' end and the 3' end in the base sequences of SEQ ID NOs: 1 to 7, or a sequence obtained by deleting a sequence of an intermediate region. The polynucleotides of SEQ ID NOs: 1 to 7 are described below. In the base sequences of SEQ ID NOs: 1 to 7 and SEQ ID NOs: 8 to 13 to be described below, the modified bases are underlined. The modified bases are described in detail in the following description.
SARS-CoV-2 spike glycoprotein-binding nucleic acid molecule 1 (SEQ ID NO:1) 
GGTATGTCTCCGCCACTGAAATCCGTGCCTAATCTCACCCCACGGAATTCATGGCA
AAGCCGAGGTGTCTTGTATTC
SARS-CoV-2 spike glycoprotein-binding nucleic acid molecule 2 (SEQ ID NO:2) GGTATGTCTCCGCCACTGAAATCTAATCTCACATTGTAAGCAAAGGAGAATAAGCA AAGCCGAGGTGTCTTGTATTC
SARS-CoV-2 spike glycoprotein-binding nucleic acid molecule 3 (SEQ ID NO: 3) 
GGTATGTCTCCGCCACTGAAATCCCTGACCGCTGACCAAATCTCAGTGCAGATGCA
AAGCCGAGGTGTCTTGTATTC
SARS-CoV-2 spike glycoprotein-binding nucleic acid molecule 4 (SEQ ID NO: 4) 
GGTATGTCTCCGCCACTGAAATCTTGTCCCCTAATAGGTTCCGACTGACAAGTGCA
AAGCCGAGGTGTCTTGTATTC
SARS-CoV-2 spike glycoprotein-binding nucleic acid molecule 5 (SEQ ID NO: 5) 
GGTTTAGCCCTCGACTCCAAATGTGCACTCTCCCGGTATCCCTAATCTCACCCGATA
CCGTTGATGTGCTGCCAATAC
SARS-CoV-2 spike glycoprotein-binding nucleic acid molecule 6 (SEQ ID NO: 6) 
GGTTTAGCCCTCGACTCCAAATGACATGAGCCAGGTGCATCTTGAACGTCATAGAT
ACCGTTGATGTGCTGCCAATAC
SARS-CoV-2 spike glycoprotein-binding nucleic acid molecule 7 (SEQ ID NO: 7)

GGTTTAGCCCTCGACTCCAAATGTGCTGATACTCGGTATCCCTAATCTCACCCGATA
CCGTTGATGTGCTGCCAATAC

The partial sequence is not particularly limited, and the partial sequence of the base sequence of SEQ ID NO: 1 is the base sequence of SEQ ID NO: 8, the partial sequence of the base sequence of SEQ ID NO: 2 is the base sequence of SEQ ID NO: 9, the partial sequence of the base sequence of SEQ ID NO: 3 is the base sequence of SEQ ID NO: 10, the partial sequence of the base sequence of SEQ ID NO: 5 is the base sequence of SEQ ID NO: 11, the partial sequence of the base sequence of SEQ ID NO: 6 is the base sequence of SEQ ID NO: 12, or the partial sequence of the base sequence of SEQ ID NO: 7 is the base sequence of SEQ ID NO: 13, for example.
SARS-CoV-2 spike glycoprotein-binding nucleic acid molecule 8 (SEQ ID NO: 8)
   CCACTGAAATCCG**T**GCC**T**AA**T**C**T**CACCCCACGGAA**TT**CATGG
SARS-CoV-2 spike glycoprotein-binding nucleic acid molecule 9 (SEQ ID NO: 9)
   TCCGCCACTGAAATC**T**AA**T**C**T**CACA**TT**G**T**AAGCAAAGGAGAA**T**AA
SARS-CoV-2 spike glycoprotein-binding nucleic acid molecule 10 (SEQ ID NO: 10)
   CCACTGAAATCCC**T**GACCGC**T**GACCAAA**T**C**T**CAG**T**GCAGA**T**
SARS-CoV-2 spike glycoprotein-binding nucleic acid molecule 11 (SEQ ID NO: 11)
   TGTGCACTCTCCCGG**T**A**T**CCC**T**AA**T**C**T**CACCCGA**T**ACC
SARS-CoV-2 spike glycoprotein-binding nucleic acid molecule 12 (SEQ ID NO: 12)
   TGACATGAGCCAGG**T**GCA**T**C**TT**GAACG**T**CA**T**AGA**T**ACCG**TT**GA**T**G**T**GC**T**G
SARS-CoV-2 spike glycoprotein-binding nucleic acid molecule 13 (SEQ ID NO: 13)
   GCTGATACTCGG**T**A**T**CCC**T**AA**T**C**T**CACCCGA**T**ACCG

Regarding the polynucleotide (b), the "sequence identity" is not particularly limited as long as, for example, it allows the polynucleotide (b) to bind to the SARS-CoV-2 spike glycoprotein. The identity is, for example, 80% or more, preferably 85% or more, more preferably 90% or more, still more preferably 95% or more, 96% or more, 97% or more, particularly preferably 98% or more, and most preferably 99% or more. The "sequence identity" may be calculated with analysis software such as BLAST or FASTA using default parameters, for example (hereinafter, the same applies).

The polynucleotide (b) may be, for example, the following polynucleotide (b1). In this case, the nucleic acid molecule of the present invention may be, for example, a molecule consisting of the polynucleotide (b1) or a molecule including the polynucleotide (b1). In the following polynucleotide (b1), the expression "including any of the base sequences of SEQ ID NOs: 8 to 13" can also be referred to as "any of the base sequences of SEQ ID NOs: 8 to 13 is conserved", for example:
(b1) a polynucleotide consisting of a base sequence having 80% or more identity to any base sequence of the polynucleotide (a), including any of the base sequences of SEQ ID NOs: 8 to 13, and binding to a SARS-CoV-2 spike glycoprotein.

The polynucleotide in the nucleic acid molecule of the present invention may be, for example, the following polynucleotide (b2). In this case, the nucleic acid molecule of the present invention may be, for example, a molecule consisting of the polynucleotide (b2) or a molecule including the polynucleotide (b2):
(b2) a polynucleotide consisting of a base sequence having 80% or more identity to any base sequence of the polynucleotide (a), capable of forming a secondary structure represented by each of the following formulae (2) to (14), and binding to a SARS-CoV-2 spike glycoprotein.

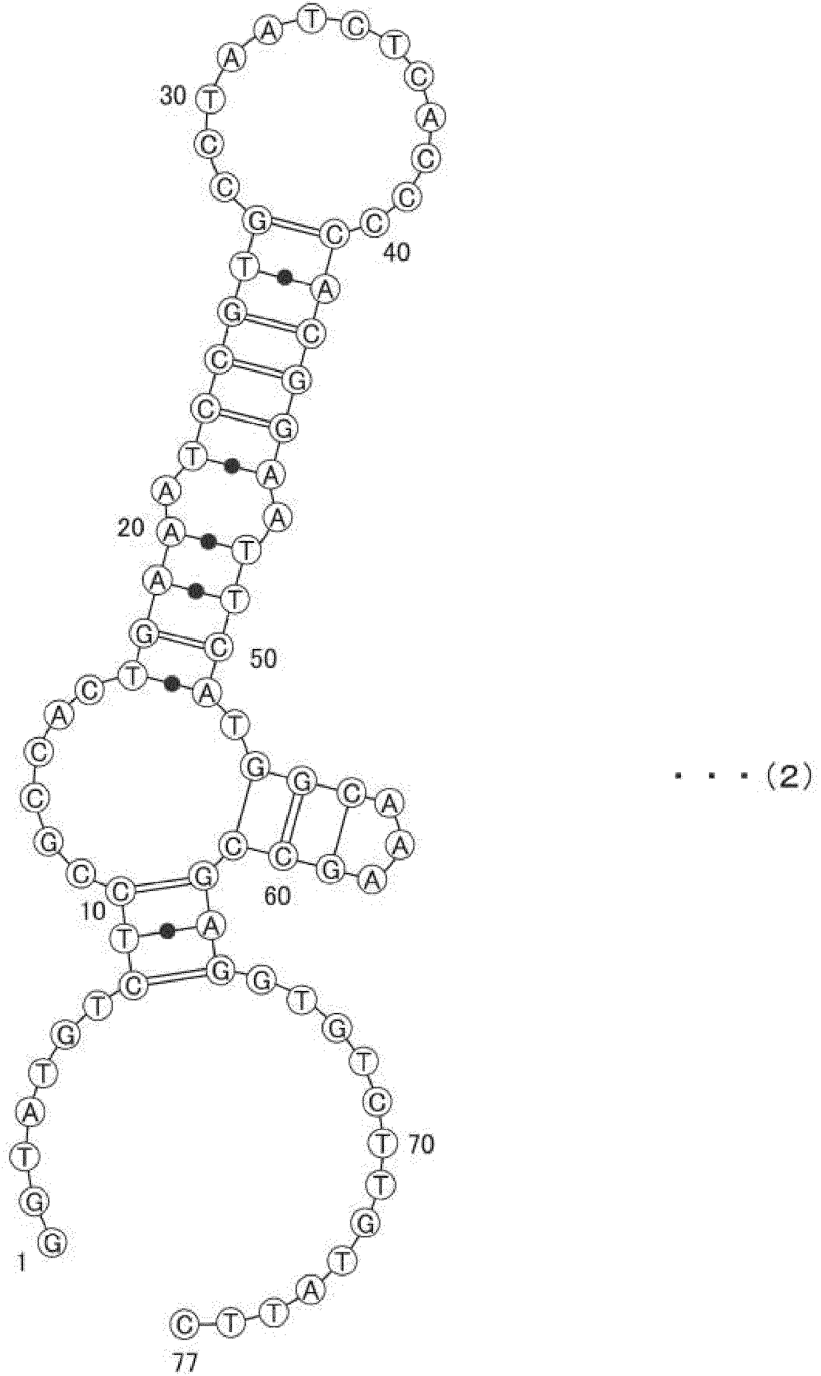

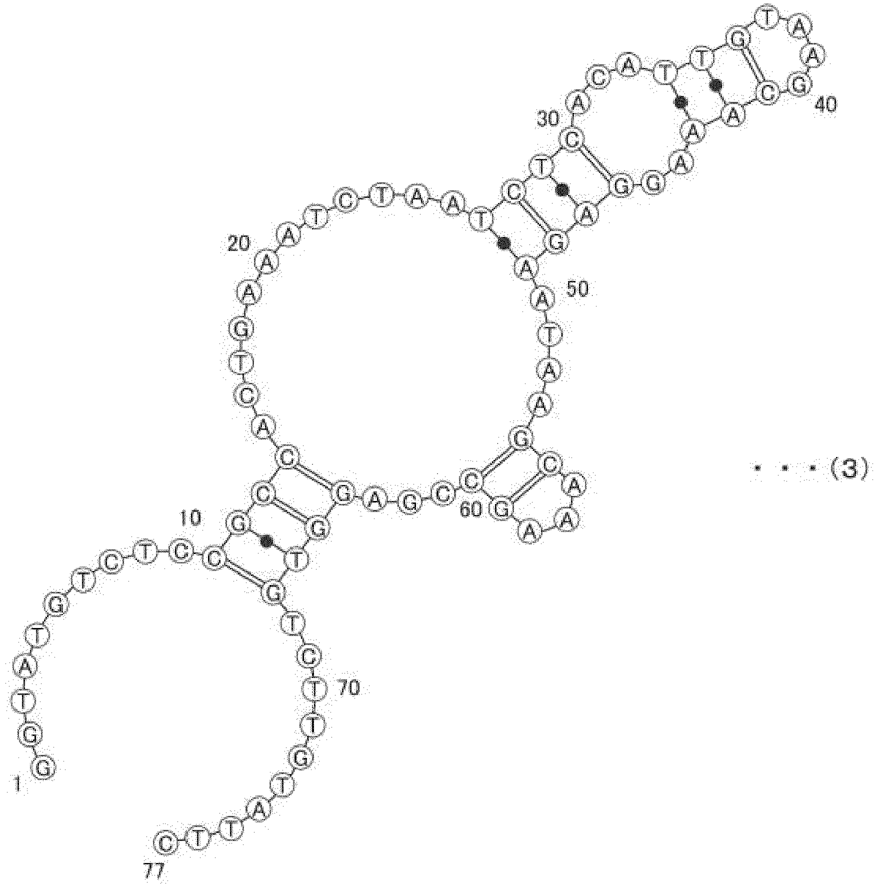

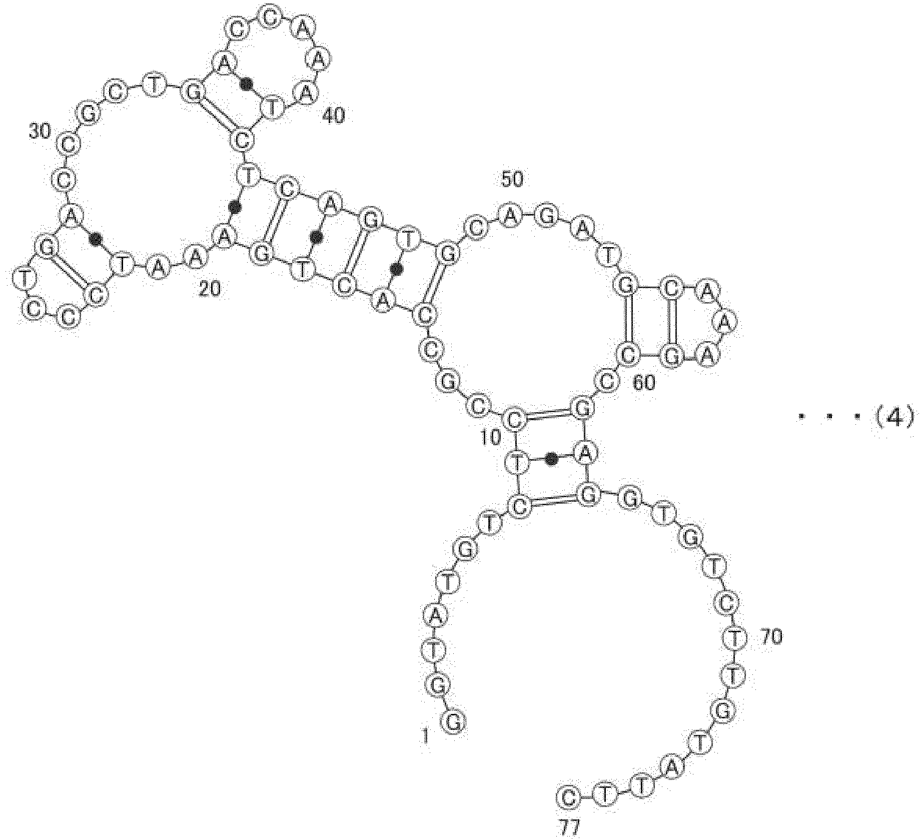

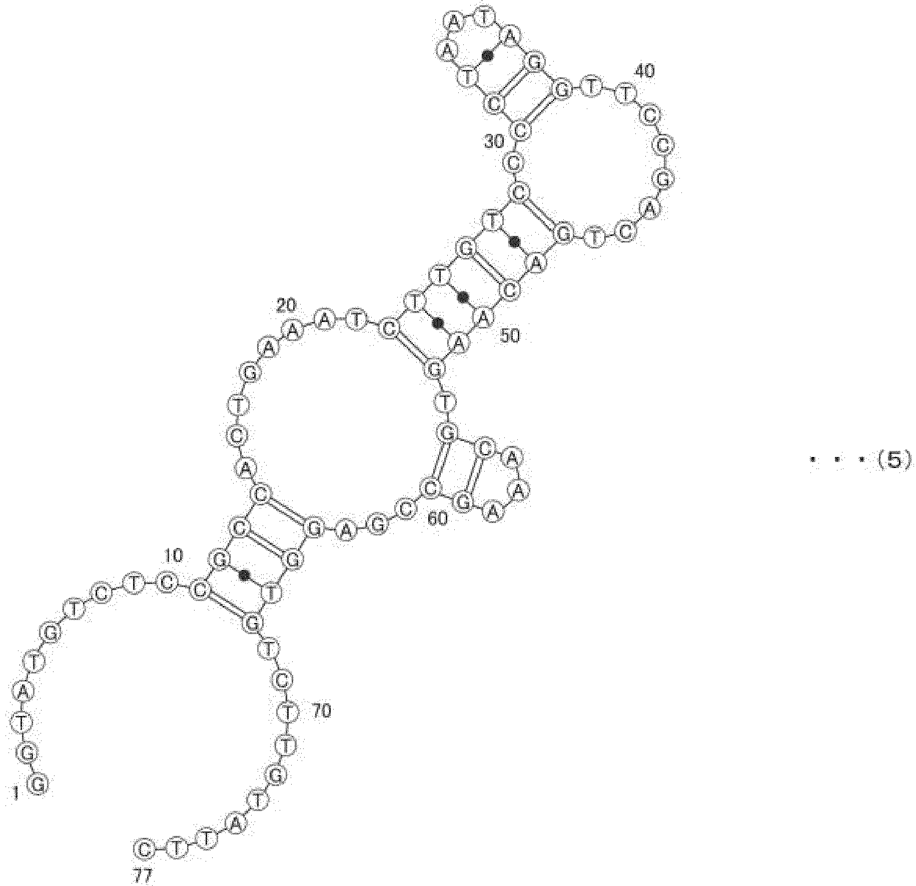

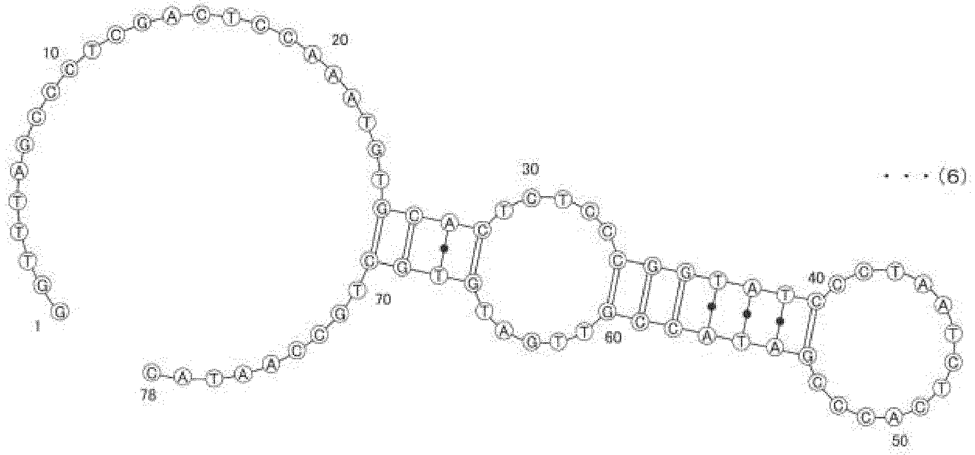

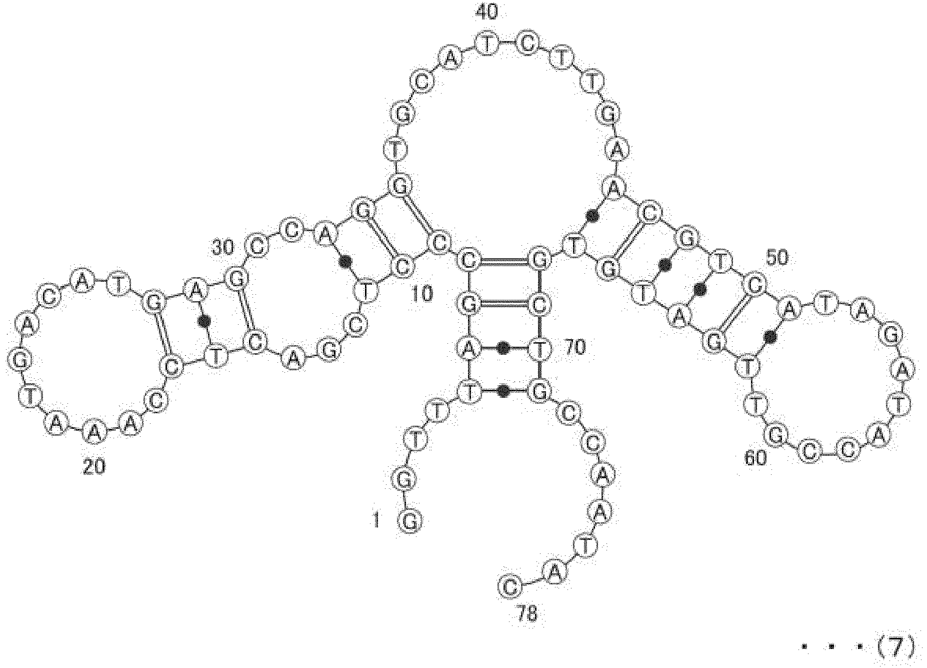

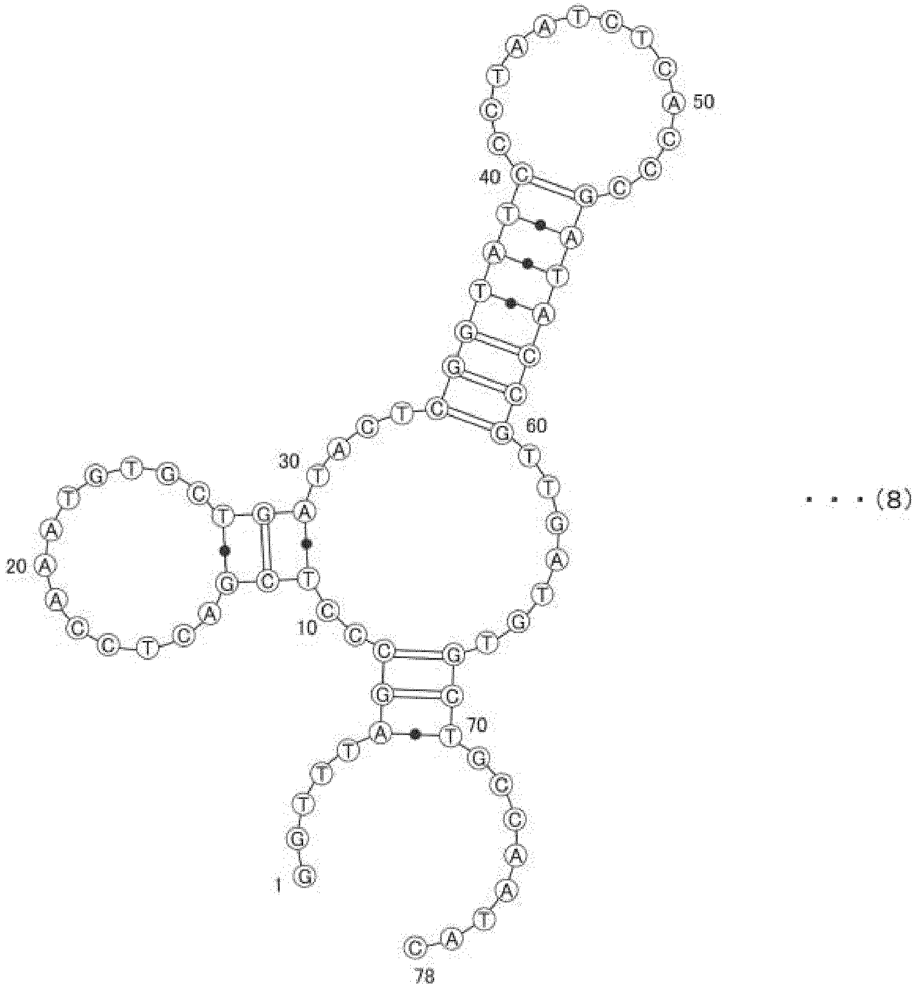

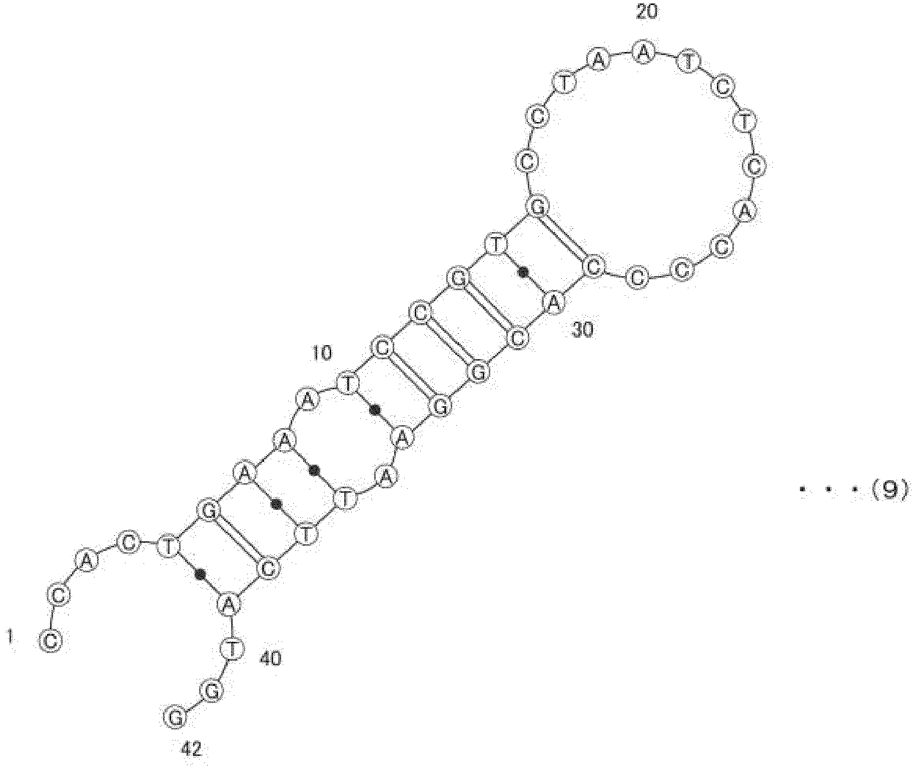

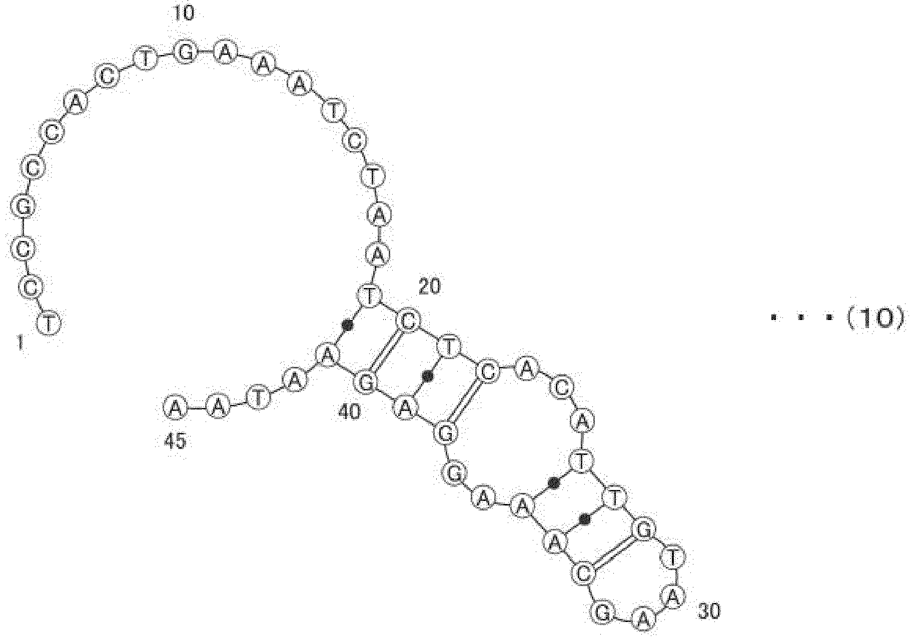

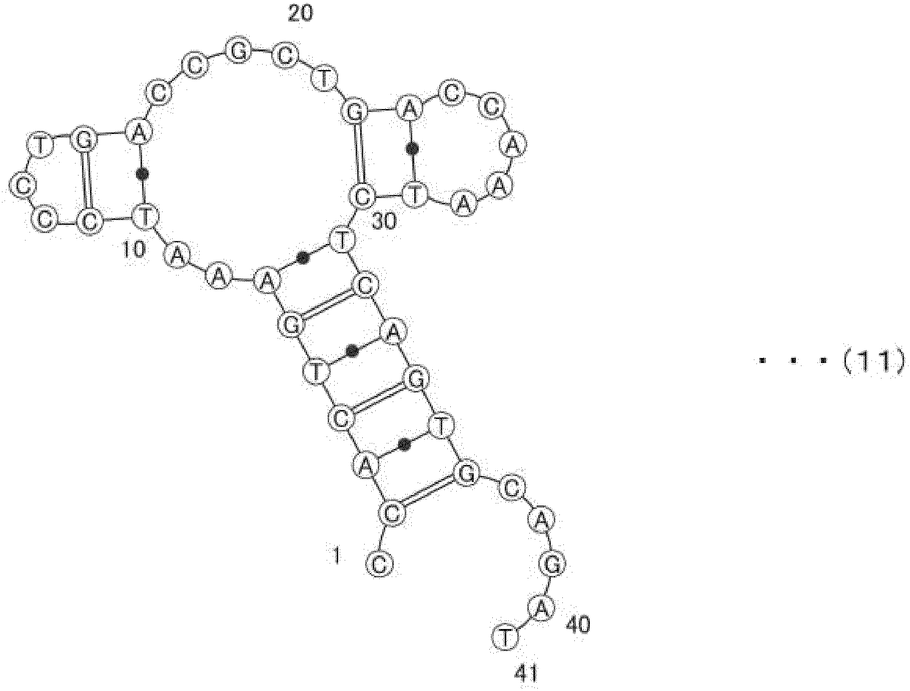

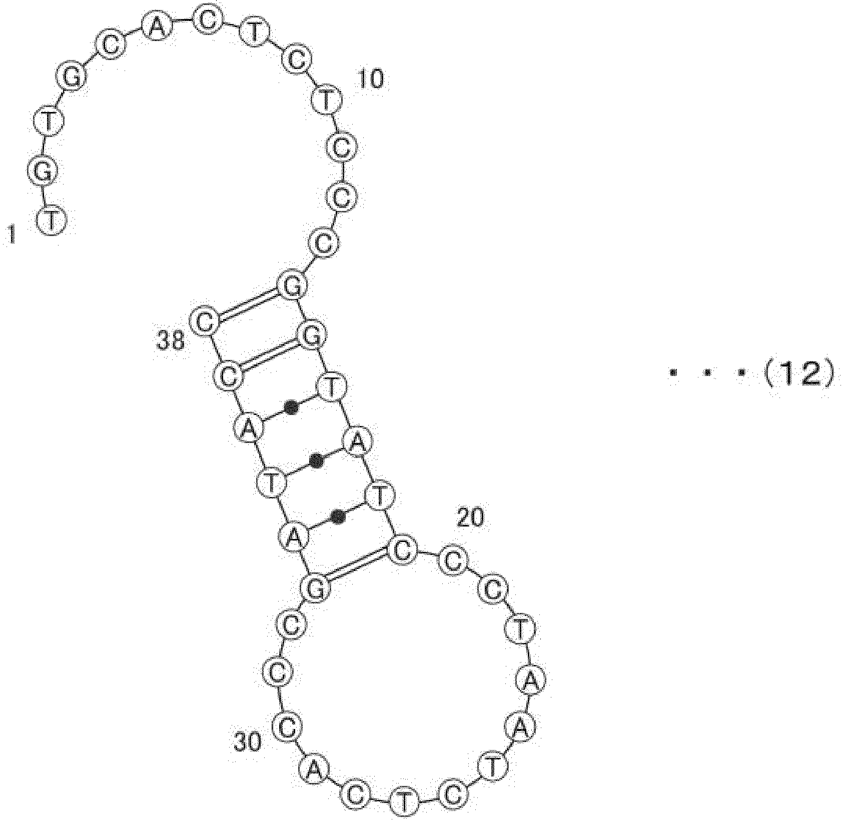

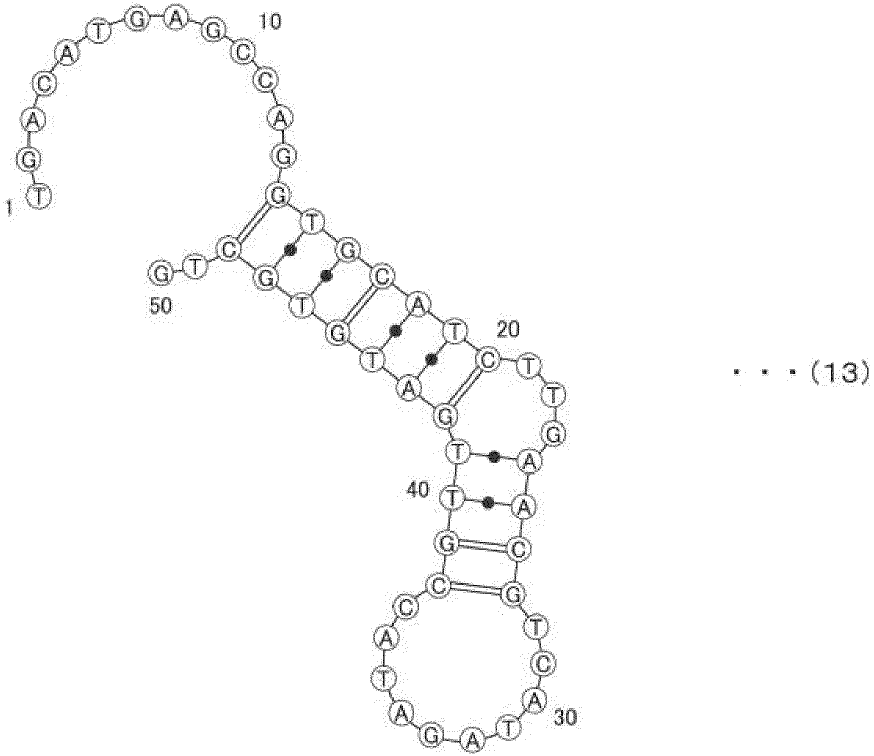

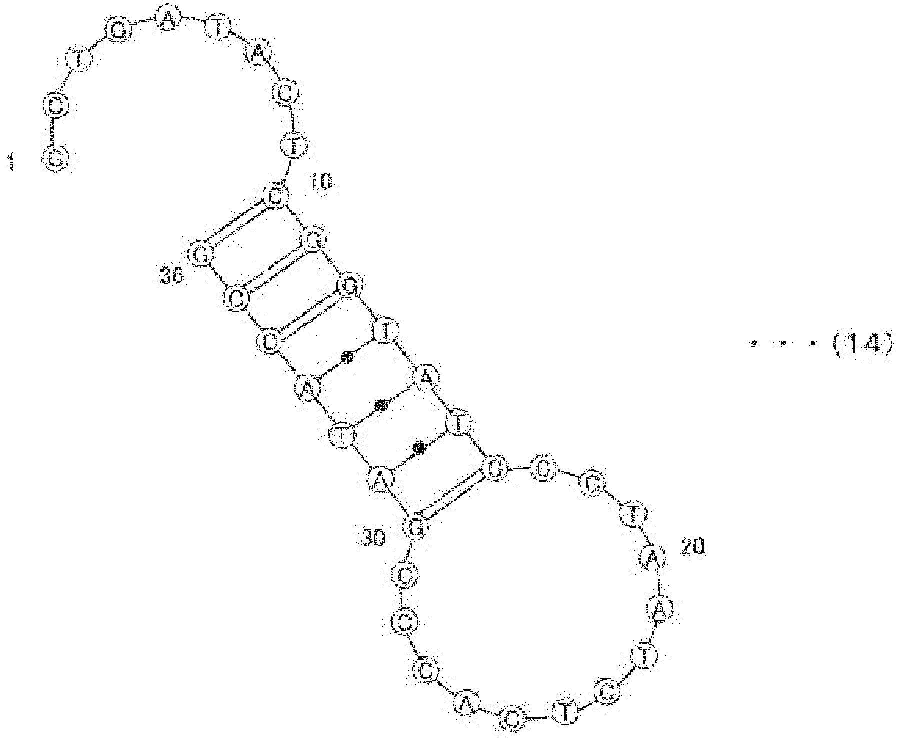

Specifically, the polynucleotide (b2) is, for example, a polynucleotide consisting of a base sequence having 80% or more identity to any of the base sequences of SEQ ID NOs: 1 to 13, capable of forming a secondary structure represented by each of the formulae (2) to (14), and binding to a SARS-CoV-2 spike glycoprotein.

Regarding the sequence identity in the polynucleotides (b1) and (b2), for example, reference can be made to the description as to the sequence identity in in the polynucleotide (b).

The correspondence relationships between the base sequences of SEQ ID NOs: 1 to 13 and the secondary structures represented by formulae (2) to (14) are shown below.

The polynucleotide consisting of the base sequence of SEQ ID NO: 1 is capable of forming, for example, a secondary structure represented by the formula (2).

The polynucleotide consisting of the base sequence of SEQ ID NO: 2 is capable of forming, for example, a secondary structure represented by the formula (3).

The polynucleotide consisting of the base sequence of SEQ ID NO: 3 is capable of forming, for example, a secondary structure represented by the formula (4).

The polynucleotide consisting of the base sequence of SEQ ID NO: 4 is capable of forming, for example, a secondary structure represented by the formula (5).

The polynucleotide consisting of the base sequence of SEQ ID NO: 5 is capable of forming, for example, a secondary structure represented by the formula (6).

The polynucleotide consisting of the base sequence of SEQ ID NO: 6 is capable of forming, for example, a secondary structure represented by the formula (7).

The polynucleotide consisting of the base sequence of SEQ ID NO: 7 is capable of forming, for example, a secondary structure represented by the formula (8).

The polynucleotide consisting of the base sequence of SEQ ID NO: 8 is capable of forming, for example, a secondary structure represented by the formula (9).

The polynucleotide consisting of the base sequence of SEQ ID NO: 9 is capable of forming, for example, a secondary structure represented by the formula (10).

The polynucleotide consisting of the base sequence of SEQ ID NO: 10 is capable of forming, for example, a secondary structure represented by the formula (11).

The polynucleotide consisting of the base sequence of SEQ ID NO: 11 is capable of forming, for example, a secondary structure represented by the formula (12).

The polynucleotide consisting of the base sequence of SEQ ID NO: 12 is capable of forming, for example, a secondary structure represented by the formula (13).

The polynucleotide consisting of the base sequence of SEQ ID NO: 13 is capable of forming, for example, a secondary structure represented by the formula (14).

In addition, the polynucleotide (b2) is specifically a polynucleotide consisting of a base sequence having 80% or more identity to any of the base sequences of SEQ ID NOs: 1 to 3 and 5 to 7, capable of forming a secondary structure represented by each of the formulae (9) to (14) respectively corresponding to SEQ ID NOs: 8 to 13, which are partial sequences of the base sequences of SEQ ID NOs: 1 to 3 and 5 to 7, respectively, and binding to a SARS-CoV-2 spike glycoprotein.

Regarding the polynucleotide (b2), the expression "capable of forming a secondary structure" means, for example, that the polynucleotide (b2) is capable of forming a stem structure and a loop structure in the formulae. The stem structure and the loop structure will be described below. In the stem structure, the opposing base pairs may be any combination of bases capable of forming hydrogen bonds. Examples of the combination of bases capable of forming hydrogen bonds include A-T, T-A, A-U, U-A, G-C, and C-G. The "secondary structure" may be, for example, a secondary structure actually formed or a secondary structure obtained by a simulation performed by known methods.

The polynucleotide in the nucleic acid molecule of the present invention may be, for example, the following polynucleotide (c). In this case, the nucleic acid molecule of the present invention may be, for example, a molecule consisting of the following polynucleotide (c) or a molecule including the following polynucleotide (c):
(c) a polynucleotide consisting of a base sequence complementary to a polynucleotide that hybridizes to a polynucleotide consisting of any base sequence of the polynucleotide (a) under a stringent condition, and binding to a SARS-CoV-2 spike glycoprotein.

Regarding the polynucleotide (c), the expression "polynucleotide that hybridizes to" is not particularly limited as long as, for example, the polynucleotide (c) is a polynucleotide perfectly or partially complementary to the polynucleotide (a) and it allows the polynucleotide (c) to bind to the SARS-CoV-2 spike glycoprotein. The hybridization can be detected, for example, by various hybridization assays. The hybridization assay is not particularly limited, and may be, for example, the method described in Sambrook et al. "Molecular Cloning: A Laboratory Manual 2nd Ed." [Cold Spring Harbor Laboratory Press (1989)].

Regarding the polynucleotide (c), the "stringent condition" may be any of a low stringent condition, a middle stringent condition, and a high stringent condition, for example. The "low stringent condition" refers to, for example, a condition in which 5 × SSC (standard saline citrate), 5 × Denhardt's solution, 0.5% SDS (sodium dodecyl sulfate), and 50% formamide are used at 32°C. The "middle stringent condition" refers to, for example, a condition in which 5 × SSC, 5 × Denhardt's solution, 0.5% SDS, and 50% formamide are used at 42°C. The "high stringent condition" refers to, for example, a condition in which 5 × SSC, 5 × Denhardt's solution, 0.5% SDS, and 50% formamide are used at 50°C. Those skilled in the art can adjust the degree of the stringency, for example, by appropriately selecting the conditions such as a temperature, a salt concentration, the concentration and the length of the probe, an ionic strength, time, and the like. The "stringent condition" may be, for example, the condition described in Sambrook et al. "Molecular Cloning: A Laboratory Manual 2nd Ed." [Cold Spring Harbor Laboratory Press (1989)].

The polynucleotide in the nucleic acid molecule of the present invention may be, for example, the following polynucleotide (d). In this case, the nucleic acid molecule of the present invention may be, for example, a molecule consisting of the polynucleotide (d) or a molecule including the polynucleotide (d):
(d) a polynucleotide consisting of a base sequence obtained by deletion, substitution, insertion, and/or addition of one to several bases in any base sequence of the polynucleotide (a), and binding to a SARS-CoV-2 spike glycoprotein.

Regarding the polynucleotide (d), the expression "one to several" is not limited as long as, for example, it allows the polynucleotide (d) to bind to the SARS-CoV-2 spike glycoprotein. The expression "one to several" may be, for example, 1 to 16, 1 to 12, 1 to 10, 1 to 8, 1 to 7, 1 to 5, 1 to 3, 1 to 2, or 1 in the base sequence of the polynucleotide (a). In the present invention, for example, the numerical range regarding the number of bases, the number of sequences, and the like discloses all the positive integers falling within that range. That is, for example, the description "1 to 5 bases" discloses all of "1, 2, 3, 4, and 5 bases" (hereinafter, the same applies).

The polynucleotide (d) may be, for example, the following polynucleotide (d1). In this case, the nucleic acid molecule of the present invention may be, for example, a molecule consisting of the polynucleotide (d1) or a molecule including the polynucleotide (d1). In the following polynucleotide (d1), the expression "including any of the base sequences of SEQ ID NOs: 8 to 13" can also be referred to as "any of the base sequences of SEQ ID NOs: 8 to 13 is conserved":
(d1) a polynucleotide consisting of a base sequence obtained by deletion, substitution, insertion, and/or addition of one to several bases in any base sequence of the polynucleotide (a), including any of the base sequences of SEQ ID NOs: 8 to 13, and binding to a SARS-CoV-2 spike glycoprotein.

The polynucleotide (d) may be, for example, the following polynucleotide (d2). In this case, the nucleic acid molecule of the present invention may be, for example, a molecule consisting of the polynucleotide (d2) or a molecule including the polynucleotide (d2).
(d2) a polynucleotide consisting of a base sequence obtained by deletion, substitution, insertion, and/or addition of one to several bases in any base sequence of the polynucleotide (a), capable of forming a secondary structure represented by each of the formulae (2) to (14), and binding to a SARS-CoV-2 spike glycoprotein.

Regarding the above description regarding the number of the expression "one to several" bases in the polynucleotides (d1) and (d2), reference can be made to the description as to the number of the "one to several" bases in the polynucleotides (d), for example.

In the nucleic acid molecule of the present invention, the structural units of the polynucleotide are, for example, nucleotide residues, examples of which include deoxyribonucleotide residues and ribonucleotide residues. The polynucleotide is, for example, a DNA consisting of a deoxyribonucleotide residue(s) or a DNA including a deoxyribonucleotide residue(s) and a ribonucleotide residue(s), and the polynucleotide may further include a nonnucleotide residue(s), as described below.

The nucleic acid molecule of the present invention may be, for example, a molecule consisting of any of the polynucleotides (a) to (d), or a molecule including any of the polynucleotides (a) to (d). In the latter case, the nucleic acid molecule of the present invention may include two or more polynucleotides of any of the polynucleotides (a) to (d) as described below, for example. The two or more polynucleotides (a) to (d) may be the polynucleotides with the same sequence or different sequences. In the latter case, the nucleic acid molecule of the present invention may further include a linker(s) and/or an additional sequence(s), for example. The linker is a sequence present between polynucleotides, for example. The additional sequence is a sequence added to an end, for example.

When the nucleic acid molecule of the present invention includes, for example, a plurality of polynucleotides selected from the above-described polynucleotides, it is preferable that the plurality of polynucleotide sequences be linked to each other to form a single-stranded polynucleotide. The plurality of polynucleotide sequences may be linked to each other directly, or may be linked to each other indirectly with a linker, for example. It is preferable that the polynucleotide sequences be linked to each other directly or indirectly at their ends. When the nucleic acid molecule of the present invention includes the plurality of polynucleotide sequences, the number of the sequences is not particularly limited, and is, for example, 2 or more, 2 to 20, 2 to 10, or 2 or 3.

The length of the linker is not particularly limited, and is, for example, 1 to 200-mer, 1 to 24-mer, 1 to 20-mer, 3 to 12-mer, or 5 to 9-mer. The structural units of the linker are, for example, nucleotide residues, examples of which include deoxyribonucleotide residues and ribonucleotide residues. The linker is not particularly limited, and examples thereof include polynucleotides such as a DNA consisting of a deoxyribonucleotide residue(s) and a DNA including a deoxyribonucleotide residue(s). Specific examples of the linker include polydeoxythymine (poly dT), poly-deoxyadenine (poly dA), and poly dAdT, which is a repetitive sequence of A and T. The linker is preferably poly dT or poly dAdT.

In the nucleic acid molecule of the present invention, the polynucleotide preferably is a single-stranded polynucleotide. It is preferable that the single-stranded polynucleotide be capable of forming a stem structure and a loop structure by self-annealing, for example. It is preferable that the polynucleotide be capable of forming a stem-loop structure, an internal loop structure, and/or a bulge structure, for example.

The nucleic acid molecule of the present invention may be a double strand, for example. When the nucleic acid molecule is a double strand, for example, one single-stranded polynucleotides includes any of the polynucleotides (a) to (d), and the other single-stranded polynucleotide is not limited. The other single-stranded polynucleotide may be, for example, a polynucleotide including a base sequence complementary to any of the polynucleotides (a) to (d). When the nucleic acid molecule of the present invention is a double strand, it is preferable to dissociate the double strand into single-stranded polynucleotides by denaturation or the like before use, for example. Also, it is preferable that the dissociated single-stranded polynucleotide including any of the polynucleotides (a) to (d) is forming a stem structure and a loop structure as described above, for example.

In the present invention, the expression "capable of forming a stem structure and a loop structure" encompasses that, for example, a stem structure and a loop structure are formed actually, and also, even if a stem structure and a loop structure are not formed, they can be formed depending on conditions. The expression "capable of forming a stem structure and a loop structure" encompasses, for example, both the cases where the formation thereof has been confirmed through an experiment and where the formation thereof is predicted through simulation using a computer or the like.

The structural units of the nucleic acid molecule of the present invention are, for example, nucleotide residues. The length of the nucleic acid molecule is not particularly limited. The lower limit of the length of the nucleic acid molecule is, for example, 15-mer, 35-mer, 55-mer, or 75-mer. The upper limit of the length of the nucleic acid molecule is, for example, 1000-mer, 200-mer, 100-mer, 90-mer, or 80-mer. The length of the nucleic acid molecule is, for example, in the range from 15 to 1000-mer, from 35 to 200-mer, from 55 to 90-mer, or from 75 to 80-mer.

Examples of the nucleotide residue include deoxyribonucleotide residues and ribonucleotide residues. The nucleic acid molecule of the present invention may be, for example, a DNA consisting of a deoxyribonucleotide residue(s) or a DNA including one to several ribonucleotide residues. In the latter case, for example, the expression "one to several" is not particularly limited and is, for example, 1 to 91, 1 to 30, 1 to 15, 1 to 7, 1 to 3, 1, or 2 in the polynucleotide.

The polynucleotide may include, as a base in a nucleotide residue, a natural base or a modified base. The natural base (non-artificial base) is not particularly limited, and may be, for example, a purine base with a purine skeleton or a pyrimidine base with a pyrimidine skeleton. The purine base is not particularly limited, and examples thereof include adenine (a) and guanine (g). The pyrimidine base is not particularly limited, and examples thereof include cytosine (c), thymine (t), and uracil (u).

When the polynucleotide includes the modified base(s), the site and the number of the modified bases are not particularly limited. When the nucleic acid molecule of the present invention has the modified base(s), one or some or all of the underlined thymine bases are modified base(s) in the polynucleotides of SEQ ID NOs: 1 to 13, for example. When the underlined thymine bases are modified base(s), the modified base is preferably a modified thymine being a modified thymine base.

The modified base is a base modified with a modifying group, for example. The base to be modified with the modifying group (also referred to simply as the "base to be modified" hereinafter) is the natural base, for example. Examples of the natural base include purine bases and pyrimidine bases. The modified base is not particularly limited, and examples thereof include modified adenine, modified guanine, modified cytosine, modified thymine, and modified uracil.

In the modified base, the base to be modified may be modified with the modifying group either directly or indirectly, for example. In the latter case, the base to be modified may be modified with the modifying group via a linker, for example. The linker is not particularly limited.

In the base to be modified, a site to be modified with the modifying group is not particularly limited. When the base is a pyrimidine base, the modified site in the pyrimidine base may be, for example, the 5-position or the 6-position, preferably the 5-position of the pyrimidine skeleton. Thymine has a methyl group bound to carbon at the 5-position. Thus, when the 5-position of the pyrimidine base is modified, for example, the modifying group may be bound to the carbon at the 5-position either directly or indirectly, or the modifying group may be bound to carbon in the methyl group bound to the carbon at the 5-position either directly or indirectly. In the case where "=O" is bound to the 4-position carbon and a group other than "-CH3" or "-H" is bound to 5-position carbon in the pyrimidine skeleton, the base can be referred to as modified thymine or modified uracil.

The modified thymine base may be a nucleotide residue represented by the following formula (1) (hereinafter, also referred to as "NG7").

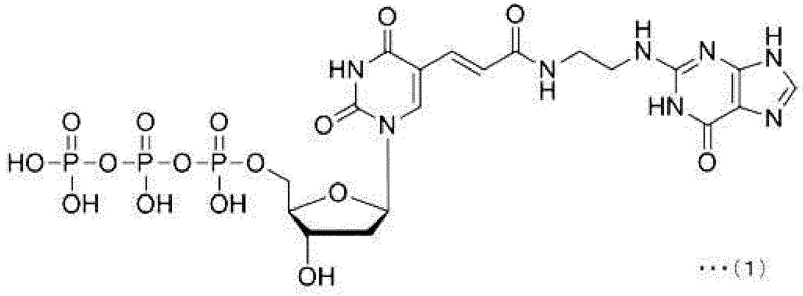

In this case, for example, a nucleotide triphosphate represented by the formula (1) can be used as a monomer molecule in the synthesis of the polynucleotide. In the synthesis of the polynucleotide, for example, the monomer molecule binds to another nucleotide triphosphate by a phosphodiester bond. The method for producing the monomer can be produced by a known method, and for example, reference can be made to WO 2018/052063.

When the modified thymine base includes a nucleotide residue represented by the formula (1), a nucleotide residue including the modified thymine base in the polynucleotide is represented by, for example, the formula (1A).

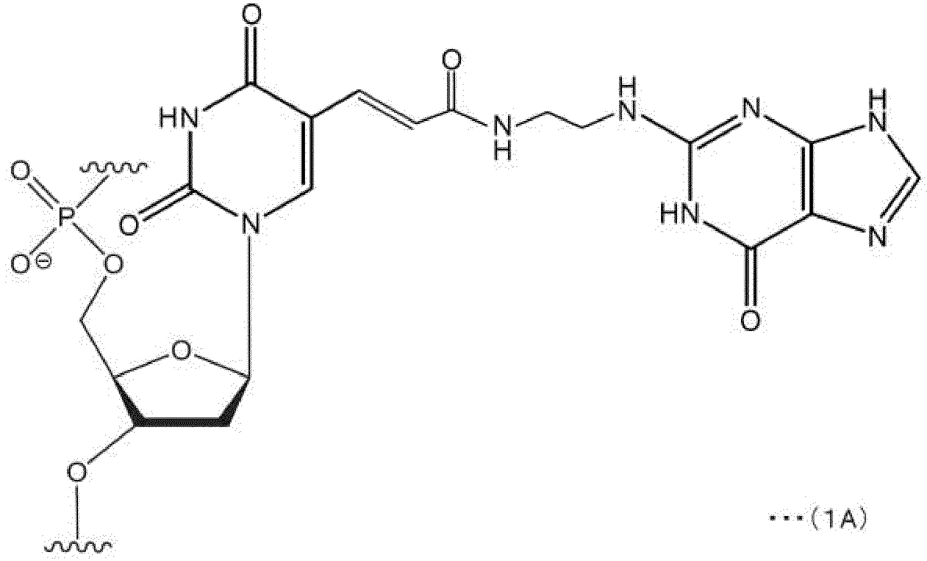

In the polynucleotide, the underlined thymine bases in the base sequences of SEQ ID NOs: 1 to 13 are preferably modified thymine bases represented by the formula (1). In this case, in the polynucleotide consisting of any of the base sequences of SEQ ID NOs: 1 to 13, the underlined nucleotide residue(s) may be a nucleotide residue(s) represented by the formulae (1A).

The polynucleotide may include one of the modified bases or two or more of the modified bases, for example.

The nucleic acid molecule of the present invention may include, for example, a modified nucleotide. The modified nucleotide may be a nucleotide including the aforementioned modified base or a nucleotide including modified sugar obtained by modifying a sugar residue, or a nucleotide including the modified base and the modified sugar.

The sugar residue is not particularly limited, and examples thereof include deoxyribose residues and ribose residues. The site to be modified in the sugar residue is not particularly limited, and may be, for example, the 2'-position or the 4'-position of the sugar residue. Either one of or both of the 2'-position and the 4'-position may be modified. Examples of the modifying group of the modified sugar include methyl groups, fluoro groups, amino groups, and thio groups.

In the case where the base is a pyrimidine base in the modified nucleotide residue, for example, it is preferable that the 2'-position and/or the 4'-position of the sugar residue is modified. Specific examples of the modified nucleotide residue include, 2'-methylated-uracil nucleotide residues, 2'-methylated-cytosine nucleotide residues, 2'-fluorinated-uracil nucleotide residues, 2'-fluorinated-cytosine nucleotide residues, 2'- aminated-uracil nucleotide residues, 2'aminated-cytosine nucleotide residues, 2'- thiolated-uracil nucleotide residues, and 2'-thiolatedcytosine nucleotide residues each obtained by modifying the 2'-position of deoxyribose residues or ribose residues.

The number of the modified bases is not particularly limited. In the polynucleotide, the number of the modified bases is, for example, one or more. The number of the modified bases in the polynucleotide is, for example, 1 to 80, 1 to 70, 1 to 50, 1 to 40, 1 to 30, 1 to 20, or 1 to 10, and all of the bases may be the modified bases. The number of the modified bases may be, for example, the number of any one of the modified bases or the total number of two or more of the modified bases. In addition, the number of the modified nucleotides in the full-length nucleic acid molecule including the polynucleotide is not particularly limited, and is, for example, 1 to 80, 1 to 50, or 1 to 20, and preferably the same as the aforementioned range.

In the polynucleotide, the proportion of the modified base is not particularly limited. The proportion of the modified base is, for example, 1/100 or more, 1/40 or more, 1/20 or more, 1/10 or more, 1/4 or more, or 1/3 or more of the total number of bases of the polynucleotide. In addition, the proportion of the modified base in the full-length nucleic acid molecule including the polynucleotide is not particularly limited, and the same as the aforementioned range. Here, the total number of bases is, for example, the sum of the number of natural bases and the number of modified bases in the polynucleotide. The proportion of the modified bases is expressed in fractions, and the total number of bases and the number of modified bases satisfying the proportion are positive integers.

When the modified base in the polynucleotide is the modified thymine, the number of the modified thymine bases is not particularly limited. In the polynucleotide, for example, natural thymine can be substituted for the modified thymine. In the polynucleotide, the number of modified thymine bases is, for example, one or more. The number of modified thymine bases may be, for example, 1 to 80, 1 to 70, 1 to 50, 1 to 40, 1 to 30, 1 to 20, or 1 to 10 in the polynuclide, and all of the thymine bases may be the modified thymine bases.

In the polynucleotide, the proportion of the modified thymine is not particularly limited. The proportion of the modified thymine is, for example, 1/100 or more, 1/40 or more, 1/20 or more, 1/10 or more, 1/4 or more, or 1/3 or more of the sum of the number of the natural thymine bases and the number of the modified thymine bases.

The nucleic acid molecule of the present invention may include, for example, one to several artificial nucleic acid monomer residues. The expression "one to several" is not particularly limited, and is, for example, 1 to 100, 1 to 50, 1 to 30, or 1 to 10 in the polynucleotide. Examples of the artificial nucleic acid monomeric residue include peptide nucleic acid (PNA), locked nucleic acid (LNA), and 2'-O, 4'-C-ethylene-bridged nucleic acid (ENA). The nucleic acid in the monomer residue is, for example, the same as described above.

It is preferable that the nucleic acid molecule of the present invention is resistant to nuclease, for example. In order to allow the nucleic acid molecule of the present invention to have nuclease resistance, it is preferable that the nucleic acid molecule of the present invention includes the modified nucleotide residue(s) and/or the artificial nucleic acid monomer residue(s), for example. Also, in order to allow the nucleic acid molecule of the present invention to have nuclease resistance, the nucleic acid molecule of the present invention may have polyethylene glycol (PEG) of several tens of kDa, deoxythymidine, or the like bound to, e.g., the 5' end or the 3' end thereof.

The nucleic acid molecule of the present invention may further include an additional sequence, for example. Preferably, the additional sequence is bound to at least one of the 5' end and the 3' end, more preferably to the 3' end of the nucleic acid molecule, for example. The additional sequence is not particularly limited. The length of the additional sequence is not particularly limited, and is, for example, 1 to 200-mer, 1 to 50-mer, 1 to 25-mer, or 18 to 24-mer. The structural unit of the additional sequence is, for example, a nucleotide residue. Examples of the nucleotide residue include deoxyribonucleotide residues and ribonucleotide residues. The additional sequence is not particularly limited, and examples thereof include polynucleotides such as DNA consisting of deoxyribonucleotide residues and DNA including ribonucleotide residues. Specific examples of the additional sequence include poly dT, and poly dA.

The nucleic acid molecule of the present invention can be used in the state where it is immobilized on a carrier, for example. It is preferable to immobilize either the 5' end or the 3' end, more preferably the 3' end of the nucleic acid molecule of the present invention, for example. When the nucleic acid molecule of the present invention is immobilized, the nucleic acid molecule may be immobilized either directly or indirectly on the carrier, for example. In the latter case, it is preferable to immobilize the nucleic acid molecule via the additional sequence, for example.

The nucleic acid molecule of the present invention may further include, for example, a labeling substance, and specifically, the labeling substance may be linked to the nucleic acid molecule. The nucleic acid molecule to which the labeling substance is linked may be referred to as, for example, a nucleic acid sensor of the present invention. The labeling substance may be linked to at least one of the 5' end and the 3' end of the nucleic acid molecule, for example. The labeling by the labeling substance may be, for example, binding or chemical modification. The labeling substance is not particularly limited, and examples thereof include enzymes, fluorescent substances, dyes, isotopes, drugs, toxins, and antibiotics. Examples of the enzyme include luciferase and NanoLuc luciferase. Examples of the fluorescent substance include fluorophores such as pyrene, TAMRA, fluorescein, Cy3 dye, Cy5 dye, FAM dye, rhodamine dye, Texas Red dye, JOE, MAX, HEX, TYE, and the like. Examples of the dye include Alexa dyes such as Alexa488, Alexa647, and the like. The labeling substance may be directly linked to the nucleic acid molecule, for example, or may be indirectly linked via a linker. The linker is not particularly limited, and is, for example, a linker of a polynucleotide or the like.

The method for producing the nucleic acid molecule of the present invention is not particularly limited. For example, the nucleic acid molecule of the present invention can be synthesized by known methods such as: nucleic acid synthesis methods utilizing chemical synthesis; and genetic engineering procedures.

The nucleic acid molecule of the present invention exhibits the ability to bind to the SARS-CoV-2 spike glycoprotein, as described above. Thus, use of the nucleic acid molecule of the present invention is not particularly limited, as long as it is the use utilizing the ability of the nucleic acid molecule to bind to the SARS-CoV-2 spike glycoprotein. The nucleic acid molecule of the present invention can be used in various methods as an alternative to, e.g., an antibody against the SARS-CoV-2 spike glycoprotein.

According to the nucleic acid molecule of the present invention, it is possible to detect a SARS-CoV-2 spike glycoprotein. Thereby, SARS-CoV-2 can be detected. The method for detecting the SARS-CoV-2 spike glycoprotein and SARS-CoV-2 is not particularly limited, and may be performed, for example, by detecting the binding between the SARS-CoV-2 spike glycoprotein and the nucleic acid with reference to the detecting method to be described below.

### (2) SARS-CoV-2 detection sensor

As described above, a detection sensor of the present invention is a SARS-CoV-2 detection sensor characterized in that it includes the nucleic acid molecule of the present invention. It is only required that the detection sensor of the present invention includes the nucleic acid molecule of the present invention, and other configurations are not particularly limited. By using the detection sensor of the present invention, the SARS-CoV-2 can be detected by, for example, causing the nucleic acid molecule to bind to the SARS-CoV-2 spike glycoprotein, as described above.

The detection sensor of the present invention may be configured so that, for example, it further includes a carrier, and the nucleic acid molecule is disposed on the carrier. Preferably, the nucleic acid molecule is immobilized on the carrier. The immobilization of the nucleic acid molecule on the carrier is as described above, for example. The method for using the detection sensor of the present invention is not particularly limited, and reference can be made to the descriptions as to the nucleic acid molecule of the present invention and the detection method of the present invention.

### (3) Detection reagent and kit

A detection reagent of the present invention is characterized in that it includes the SARS-CoV-2 spike glycoprotein-binding molecule of the present invention. It is only required that the detection reagent of the present invention includes the nucleic acid molecule of the present invention, and other configurations are by no means limited. By using the detection reagent of the present invention, it is possible to perform the detection and the like of SARS-CoV-2 as described above, for example.

The detection reagent of the present invention may include the sensor of the present invention as the nucleic acid molecule of the present invention, for example. The detection reagent of the present invention may further include a labeling substance, for example, and the labeling substance may be linked to the nucleic acid molecule. Regarding the labeling substance, for example, reference can be made to the description as to the nucleic acid molecule of the present invention. In addition, the detection reagent of the present invention may include, for example, a carrier, and the nucleic acid molecule may be immobilized on the carrier. Regarding the carrier, for example, reference can be made to the description as to the nucleic acid molecule of the present invention.

The detection reagent of the present invention may further include any component(s) in addition to the nucleic acid molecule of the present invention, for example. Examples of the component include a nonspecific adsorbent, the above-described carrier, a buffer solution, and instructions for use. Examples of the nonspecific adsorbent include dextrans, transfer RNA (tRNA), and salmon sperm DNA. As the tRNA, for example, tRNA from E. coli MRE 600 (Cat No.:10109541001, Life Science) or the like can be used. As the salmon sperm DNA, for example, deoxyribonucleic acid, low molecular weight from salmon sperm ( Cat No.:31149-10G-F, Sigma-Aldrich Co.) and the like can be used.

In the detection reagent of the present invention, for example, the nucleic acid molecule and other components such as the buffer solution may be contained in separate containers or may be contained in the same container in a mixed state or an unmixed state. When the nucleic acid molecule and the other components are contained in separate containers, the detection reagent of the present invention may be referred to as a detection kit.

### (4) Detection method

The detection method of the present invention includes, as described above, detecting a SARS-CoV-2 spike glycoprotein in the sample by bringing a sample into contact with a nucleic acid molecule; wherein the nucleic acid molecule is the SARS-CoV-2 spike glycoprotein-binding nucleic acid molecule of the present invention, and in the detecting, the SARS-CoV-2 spike glycoprotein in the sample is bonded to the nucleic acid molecule to detect the SARS-CoV-2 spike glycoprotein in the sample. The detection method of the present invention is characterized in that the nucleic acid molecule of the present invention is used, and other steps and conditions are not particularly limited. In the detection method of the present invention, the SARS-CoV-2 detection sensor of the present invention or the detection reagent or the detection kit of the present invention may be used as the nucleic acid molecule of the present invention.

According to the present invention, since the nucleic acid molecule of the present invention specifically binds to a SARS-CoV-2 spike glycoprotein, the SARS-CoV-2 spike glycoprotein and SARS-CoV-2 in a sample can be specifically detected, for example, by detecting the binding between the SARS-CoV-2 spike glycoprotein and the nucleic acid molecule. Specifically, the detection method of the present invention can analyze the presence or absence or the amount of a SARS-CoV-2 spike glycoprotein in a sample, for example, it can be said that the detection method of the present invention also can perform qualitative or quantitative analysis of SARS-CoV-2.

In the present invention, the sample is not particularly limited. Examples of the sample include aerosol, saliva, urine, plasma, and serum. The aerosol refers to, for example, a mixture of fine liquid or solid particles suspended in a gas and a surrounding gas. When the sample is an aerosol, for example, a gas that may contain the minute liquid or solid particles may be used as the sample.

The sample may be, for example, a gas sample (including the aerosol), a liquid sample, or a solid sample. The sample preferably is a liquid sample from the viewpoint of ease of handling because the liquid sample can be brought into contact with the nucleic acid molecule more easily, for example. In the case of the gas sample and the solid sample, a liquid mixture, a liquid extract, a solution, or the like of the gas sample and the solid sample prepared using a solvent may be used, for example. The solvent is not particularly limited, and may be, for example, water, physiological saline, or a buffer.

The detecting includes, for example: contacting the sample with the nucleic acid molecule to bind a SARS-CoV-2 spike glycoprotein in the sample to the nucleic acid molecule; and detecting the binding between the SARS-CoV-2 spike glycoprotein and the nucleic acid molecule. The detecting may further include, for example, detecting the presence or absence or the amount of the SARS-CoV-2 spike glycoprotein in a sample on the basis of the result obtained in the detecting.

In the contacting, the method for bringing the sample into contact with the nucleic acid molecule is not particularly limited. The contact between the sample and the nucleic acid molecule preferably is achieved in a liquid, for example. The liquid is not particularly limited, and examples thereof include water, physiological saline, and buffer solutions.

In the contacting, the conditions under which the contact between the sample and the nucleic acid molecule is caused are not particularly limited. The contact temperature is, for example, 4 to 37°C or 18 to 25°C, and the contact time is, for example, 10 to 120 minutes or 30 to 60 minutes.

In the contacting, the nucleic acid molecule may be an immobilized nucleic acid molecule immobilized on a carrier or an unimmobilized nucleic acid molecule in a free state, for example. In the latter case, the nucleic acid molecule is brought into contact with the sample in a container, for example. The nucleic acid molecule preferably is the immobilized nucleic acid molecule from the viewpoint of favorable handleability, for example. The carrier is not particularly limited, and may be, for example, a substrate, beads, or a container. The container may be a microplate or a tube, for example. The immobilization of the nucleic acid molecule is as described above, for example.

The binding-detecting is detecting the binding between the SARS-CoV-2 spike glycoprotein and the nucleic acid in the sample, as described above. By detecting whether or not the SARS-CoV-2 spike glycoprotein is bound to the nucleic acid in the sample, it is possible to detect the presence or absence of the SARS-CoV-2 spike glycoprotein in the sample (qualitative analysis), for example. Also, by detecting the degree of the binding (the amount of the binding) between the SARS-CoV-2 spike glycoprotein and the nucleic acid in the sample, it is possible to analyze the amount of the SARS-CoV-2 spike glycoprotein in the sample (quantitative analysis), for example.

In the case where the binding between the SARS-CoV-2 spike glycoprotein and the nucleic acid molecule cannot be detected, it can be determined that no SARS-CoV-2 spike glycoprotein or SARS-CoV-2 is present in the sample. In the case where the binding is detected, it can be determined that the SARS-CoV-2 spike glycoprotein and the SARS-CoV-2 are present in the sample.

The method for detecting the binding between the SARS-CoV-2 spike glycoprotein and the nucleic acid molecule is not particularly limited. A conventionally known method for detecting the binding between substances may be employed as the method, for example. Specifically, the above-described SPR may be employed, for example. Detection of the binding may be detection of a complex of the SARS-CoV-2 spike glycoprotein and the nucleic acid molecule.

### (5) SARS-CoV-2 virus deactivator

The SARS-CoV-2 virus deactivator of the present invention includes the SARS-CoV-2 spike glycoprotein-binding nucleic acid molecule of the present invention, as described above. The deactivator of the present invention is characterized in that the nucleic acid molecule of the present invention is used, and other configurations are not particularly limited.

The term "activity" refers to, for example, the functions of the SARS-CoV-2 spike glycoproteins and the SARS-CoV-2, and specifically may be, for example, the function of binding to targets (e.g., angiotensin-converting enzyme 2 (ACE2)). The term "activity" may also be, for example, infectious and toxic of the SARS-CoV-2. The term "deactivation" (also referred to as inactivation) refers to, for example, inhibiting the functions of the SARS-CoV-2 spike glycoprotein and the SARS-CoV-2. The term "deactivation" may be complete or partial inhibition of the activity.

According to the present invention, since the nucleic acid molecule of the present invention specifically binds to a SARS-CoV-2 spike glycoprotein, the SARS-CoV-2 spike glycoprotein and the SARS-CoV-2 can be deactivated, for example, by the binding between the SARS-CoV-2 spike glycoprotein and the nucleic acid molecule. The deactivation or inactivation can also be referred to as "neutralization", for example. Therefore, the deactivator of the present invention can also be referred to as a neutralizer.

### Examples

The examples of the present invention are described below. The present invention, however, is not limited by the following examples. Commercially available reagents are used according to the protocols of the reagents, unless otherwise noted.

### [Example 1]

The ability of the SARS-CoV-2 spike glycoprotein-binding nucleic acid molecule of the present invention to bind to a SARS-CoV-2 spike glycoprotein was examined by SPR.

### Aptamer

Polynucleotides consisting of the base sequences of SEQ ID NOs: 1 to 7, respectively (SARS-CoV-2 spike glycoprotein-binding nucleic acid molecules 1 to 7 (hereinafter, also referred to as "binding nucleic acid molecules 1 to 7")) were synthesized and used as DNA aptamers of the Examples. In the SARS-CoV-2 spike glycoprotein-binding nucleic acid molecules 1 to 7, the underlined thymine nucleotide residues of the base sequences of SEQ ID NOs: 1 to 7 were defined as NG7 represented by the formula (1).

The SARS-CoV-2 spike glycoprotein-binding nucleic molecules 1 to 7 were each added at the 3' end with a polydeoxyadenine (poly dA) of 20-mer and used as poly-added aptamers in the SPR described below.

### (2) Sample

Four commercially available reagents shown in Table 1 below were used for the following tests. Regarding the targets in Table 1, the "RBD domain" indicates a SARS-CoV-2 domain constituting a RBD spike glycoprotein (hereinafter, also referred to as RBD), the "S1+S2 domain" indicates a S1+S2 domain constituting a SARS-CoV-2 spike glycoprotein (hereinafter, also referred to as S1S2), the "S1 domain" indicates a S1 domain constituting a SARS-CoV-2 spike glycoprotein (hereinafter, also referred to as S1), and the "trimer" indicates a trimer of S1+S2 domain (hereinafter, also referred to as trimer). The amino acid sequences of the respective targets are prepared based on the amino acid sequences registered under the following NCBI accession numbers, and have the following amino acid sequences of SEQ ID NOs: 15 to 18.
RBD: YP_009724390.1
S1+S2 domain: YP_009724390.1
S1 domain: QHD43416.1
Trimer: QHD43416.1

**[Table 1]**

| Target | Trade name | Manufacturer | Commodity code |
|---|---|---|---|
| RBD domain | SARS-CoV-2 (2019-nCoV) Spike RBD Recombinant Protein | Sino Biological | 40592-VNAH |
| S1+S2 domain | SARS-CoV-2 (2019-nCoV) Spike S 1+S2 ECD-His Recombinant Protein | Sino Biological | 40589-V08B1 |
| S1 domain | SARS-CoV-2 (2019-nCoV) Spike S 1+S2 ECD-His Recombinant Protein | Elabscience | PKSR030481 |
| Trimer | Recombinant 2019-nCoV S-trimer Protein (His Tag) | Elabscience | PKSR030489 |

The reagents were dissolved in sterile distilled water to achieve 1 mg/ml, and the solutions were used as samples. In the analysis of the binding ability and the like described below, a SB1T buffer was used for diluting the samples. The composition of the SB1T buffer was as follows: 40 mmol/l HEPES (pH 7.4), 125 mmol/l NaCl, 1 mmol/l MgCl₂, 5 mmol/l KCl, and 0.01% Tween^{®} 20.

### (3) Analysis of binding ability by SPR

The analysis of the binding ability was carried out using a ProteOn XPR36 (BioRad) in accordance with its instructions for use.

First, as a sensor chip designed specifically for the ProteOn, a streptavidin-immobilized chip (trade name: ProteOn NLC Sensor Chip, BioRad) was set in the ProteOn XPR36. Biotinylated poly(dT) at 2.5 µmol/l was injected to a flow cell of the sensor chip using ultrapure water (DDW), and the binding was allowed to proceed until the signal intensity (RU: Resonance Unit) was saturated. The biotinylated poly(dT) was prepared by biotinylating the 5' end of 20-mer deoxythymidine. Then, the poly(dA)-added nucleic acid molecules 1 to 7 at 200 nmol/l were each injected to the flow cell of the chip using a SB1T buffer at a flow rate of 25 µl/min for 80 seconds, and the binding was allowed to proceed until the signal intensity was saturated. Subsequently, the sample was injected using the SB1T buffer at a flow rate of 50 µl/min for 120 seconds, followed by washing performed by flowing the SB1T buffer under the same conditions for 300 seconds. The signal intensity after injection of the sample was measured with the injection start of the sample being considered as 0 seconds. The SPR was carried out at 25°C.

In addition, the signal intensity was measured in the same manner as described above, except that a sample containing BSA (Sigma,catalogue number: #A7906) was used instead of the samples shown in Table 1.

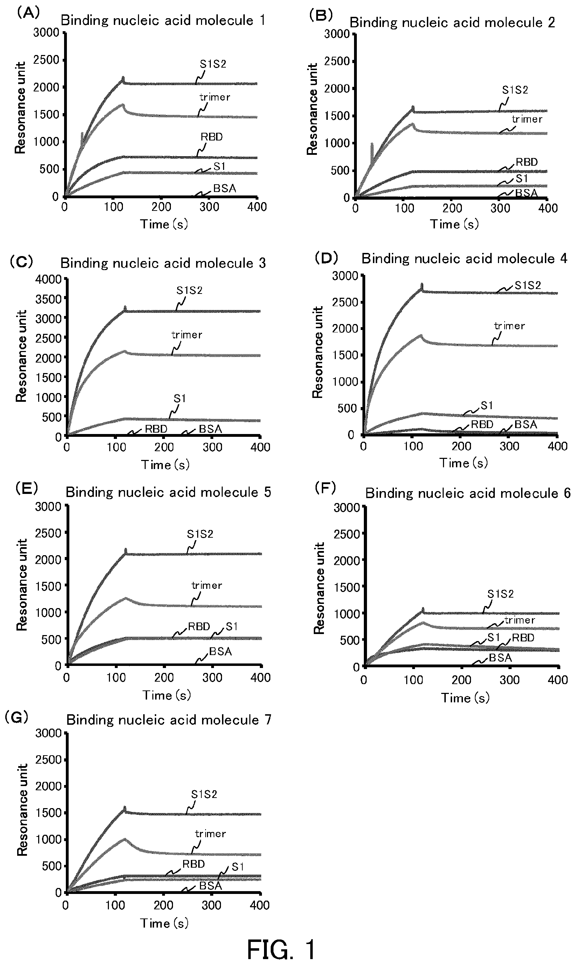

The results are shown in FIG. 1. (A) to (G) of FIG. 1 are graphs showing the ability of the nucleic acid molecules 1 to 7 to bind to the samples of 400 nmol/l. In each graph of FIG. 1, the horizontal axis indicates the elapsed time (sec) after the start of injection of the sample, and the vertical axis indicates the signal intensity (RU). As shown in (A), (B), and (E) to (G) of FIG. 1, the binding nucleic acid molecules 1, 2, and 5 to 7 exhibited ability to bind to the RBD, the S1S2, the S1, and the trimer in particular among the samples. In addition, as shown in (C) and (D) in FIG. 1, the binding nucleic acid molecules 3 and 4 exhibited ability to bind to the S1S2, the S1, and the trimers in particular among the samples. On the other hand, all of the binding nucleic acid molecules 1 to 7 had a signal intensity of approximately 0 and showed no ability to bind to the control (BSA). These results showed that the binding nucleic acid molecules 1 to 7 can detect the binding by binding to the samples with excellent specificity and measuring the signal intensities.

As described above, the ability of the binding nucleic acid molecule of the present invention to bind to the SARS-CoV-2 spike glycoprotein was verified by SPR.

### [Example 2]

The ability of the SARS-CoV-2 spike glycoprotein-binding nucleic acid molecule of the present invention to bind to the SARS-CoV-2 spike glycoprotein under the condition in a solution containing dextran, which is effective in preventing non-specific binding of nucleic acids, was examined by SPR. In addition, the dynamic parameters were obtained.

### (1) Aptamer

In addition to the polynucleotides consisting of the base sequences of SEQ ID NOs: 1 to 7, polynucleotides consisting of the base sequences of SEQ ID NOs: 8 to 13 (SARS-CoV-2 spike glycoprotein-binding nucleic acid molecules 8 to 13 (hereinafter, also referred to as binding nucleic acid molecules 8 to 13)) were synthesized in the same manner as in Example 1.

In addition, the experiment was carried out in the same manner as described above by using a control nucleic acid molecule (SEQ ID NO: 14) consisting of the base sequence of the following SEQ ID NO: 14 instead of the binding nucleic acid molecules 1 to 13. The base sequence of the following SEQ ID NO: 14 is the base sequence described in Non-Patent Literature 1 (Song Y et al, Anal. Chem. (2020), 92, 9895-9900.).

Control nucleic acid molecule (SEQ ID NO: 14)
CAGCACCGACCTTGTGCTTTGGGAGTGCTGGTCCAAGGGCGTTAATGGACA

### (2) Sample

The RBD and trimer shown in Table 1 of Example 1 were used as samples.

### (3) Analysis of binding ability by SPR

The analysis of the binding ability by SPR was carried out in the same manner as in Example 1. The concentrations of the samples were 400, 200, 100, 50, and 25 nmol/l for RBD and 200, 100, 50, 25, and 12.5 nmol/l for the trimer, respectively.

The experiment was carried out in the same manner as in Example 1 except that a buffer containing dextran (dextran-containing SB1T buffer) was used instead of the SB1T buffer. The composition of the dextran-containing SB1T buffer was as follows: 40 mmol/l HEPES (pH 7.4), 125 mmol/l NaCl, 1 mmol/l MgCh, 5 mmol/l KCl, 0.01% Tween^{®} 20, and 0.1 mmol/l dextran.

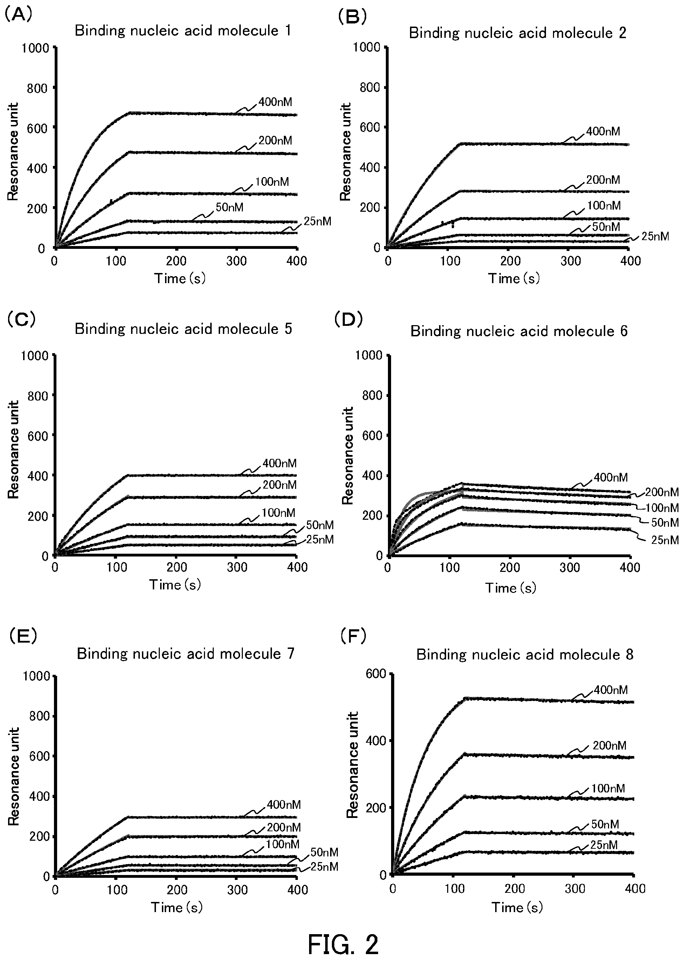

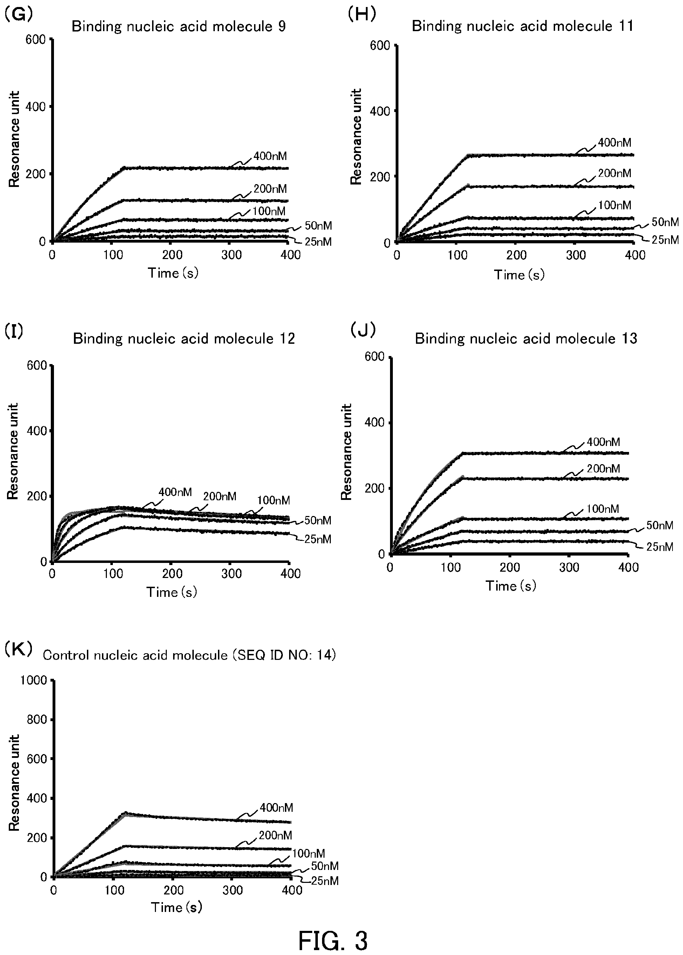

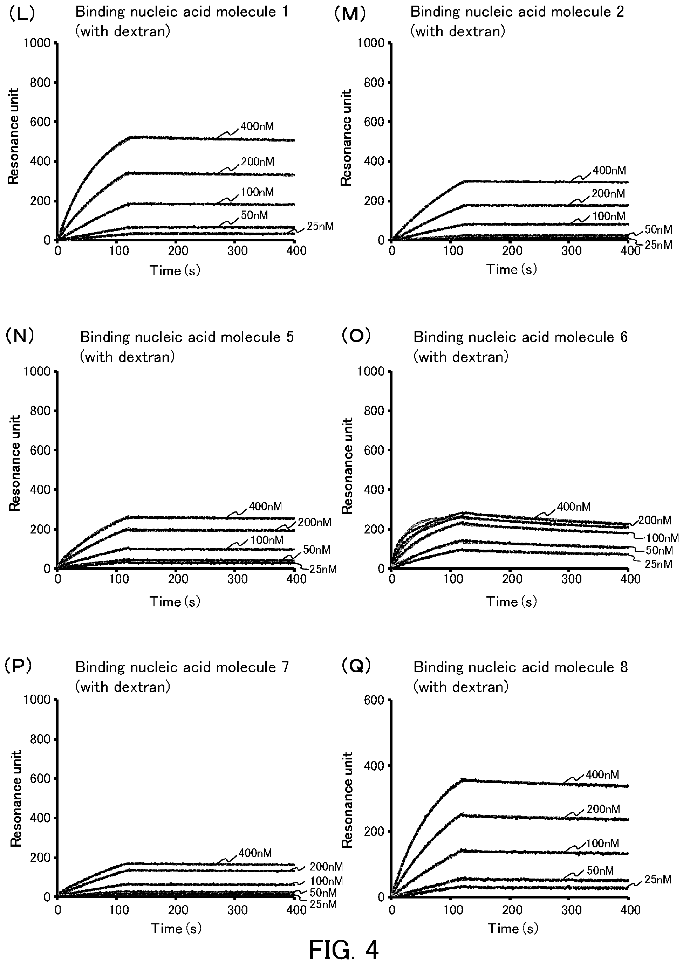

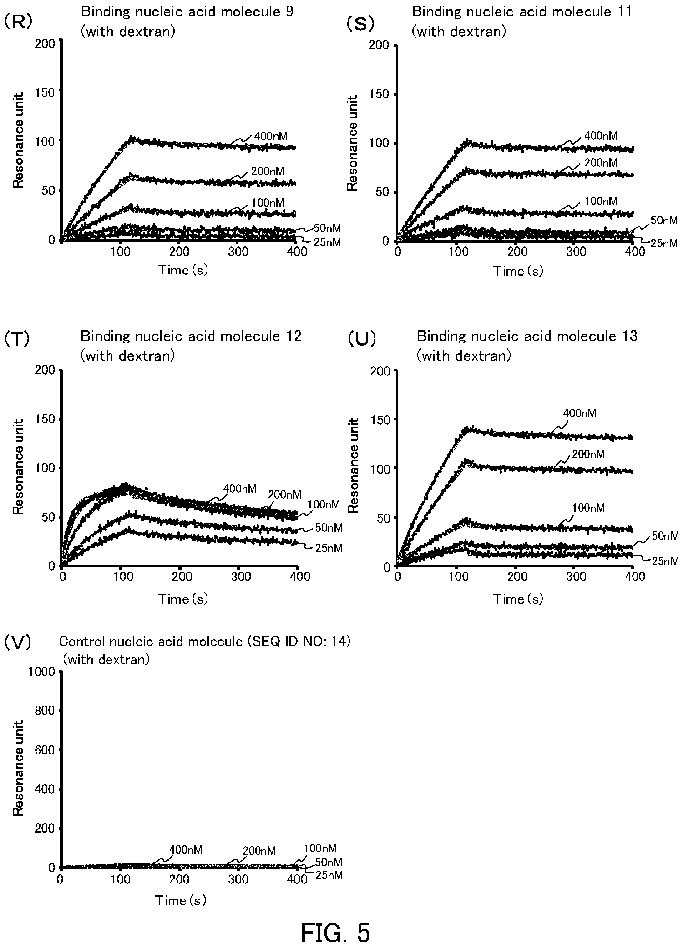

The results are shown in FIGs. 2 to 6. FIGs. 2 to 5 are graphs showing the ability of the binding nucleic acid molecules 1, 2, 5 to 9, and 11 to 13 to bind to the RBD, wherein (A) to (K) show the results without dextran and (L) to (V) show the results with dextran. In each of the graphs of FIGs. 2 to 5, the horizontal axis indicates the elapsed time (sec) after the start of injection of the sample and the vertical axis indicates the signal intensity (RU). In each of the graphs of FIGs. 2 to 6, the black dotted line indicates an actual measurement value, and the gray solid line indicates a fitting curve calculated based on the actual measurement value. As shown in (A) to (J) and (L) to (U) of FIGs. 2 to 5, the binding nucleic acid molecules 1, 2, 5 to 9, and 11 to 13 showed high ability to bind to the RBD under the conditions without dextran and with dextran. As shown in (K) of FIG. 3 and (V) of FIG. 5, the control nucleic acid molecule (SEQ ID NO: 14) showed the ability to bind to the RBD in a buffer without dextran, but lost the ability to bind to the RBD in a buffer containing dextran. These results showed that the binding nucleic acid molecules 1, 2, 5 to 9, and 11 to 13 bind to the RBD with great specificity as compared to the control nucleic acid molecule (SEQ ID NO: 14).

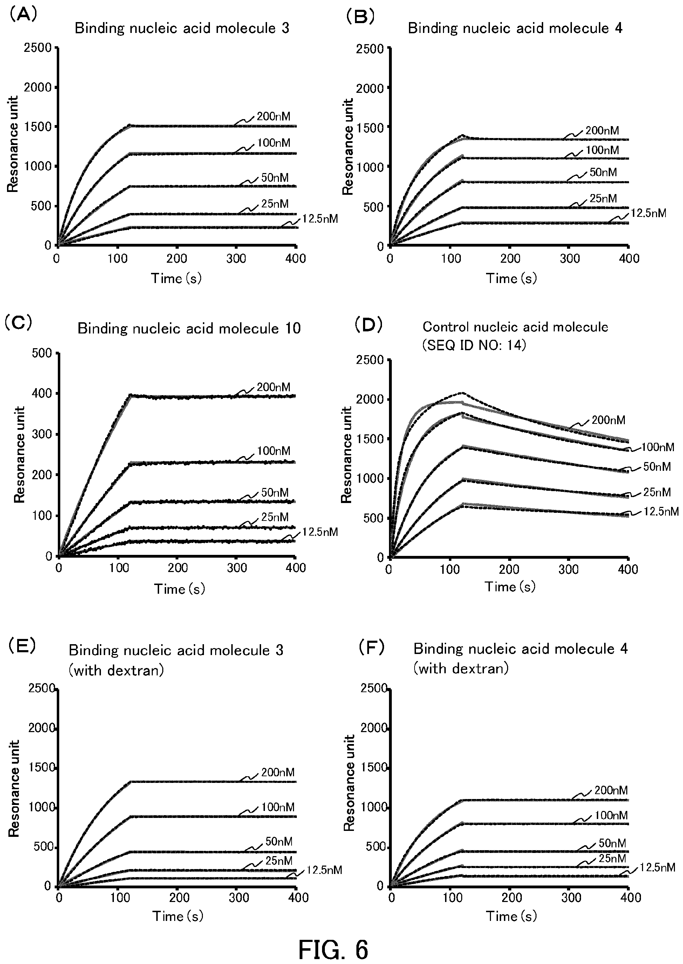

FIG. 6 shows graphs showing the ability of the binding nucleic acid molecules 3, 4, and 10 to bind to the trimer, wherein (A) to (D) show the results without dextran, and (E) and (F) show the results with dextran. In each of the graphs of FIG. 6, the horizontal axis indicates the elapsed time (sec) after the start of injection of the sample and the vertical axis indicates the signal intensity (RU). As shown in (A) to (C), (E), and (F) of FIG. 6, the binding nucleic acid molecules 3, 4, and 10 had constant signal intensity (RU) after the start of injection under the conditions without dextran and with dextran. In contrast, as shown in (D) of FIG. 6, the signal intensity (RU) of the control nucleic acid molecule (SEQ ID NO: 14) decreased with the elapsed time after the start of injection. These results showed that the binding nucleic acid molecules 3, 4, and 10 showed a slow dissociation (slow off rate) and a high binding ability to the trimer as compared to the control nucleic acid molecule (SEQ ID NO: 14).

In addition, kinetic parameters were calculated from the SPR analyses shown in FIGs. 2 to 6. The results are shown in Tables 2 and 3. Tables 2 and 3 show the dissociation constant (KD) of the binding nucleic acid molecules 1 to 13 relative to the RBD and the trimer, respectively. In Table 2, the typographical symbol "*" indicates that the dissociation constant (KD) was less than or equal to the detection limit that can be measured by SPR. This is considered to be because the dissociation of the binding nucleic acid was slow and off rate was very small. As shown in Tables 2 and 3, the binding nucleic acid molecules 1 to 13 were found to have a very low dissociation constant (KD) relative to the RBD and the trimer and show a superior binding ability as compared to the control nucleic acid molecule (SEQ ID NO: 14).

**[Table 2]**

| Nucleic acid molecule | Kd (nM) (without dextran) | Kd (nM) (with dextran) |
|---|---|---|
| Binding nucleic acid molecule 1 | 1.2 | 3.2 |
| Binding nucleic acid molecule 2 | 1.7 | 3.5 |
| Binding nucleic acid molecule 5 | Smaller than 0.1 nM * | 2.8 |
| Binding nucleic acid molecule 6 | 3.1 | 6.7 |
| Binding nucleic acid molecule 7 | Smaller than 0.1 nM * | 5.5 |
| Binding nucleic acid molecule 8 | 2 | 5.3 |
| Binding nucleic acid molecule 9 | 2.1 | 20.8 |
| Binding nucleic acid molecule 11 | Smaller than 0.1 nM * | 16.6 |
| Binding nucleic acid molecule 12 | 2.4 | 7.4 |
| Binding nucleic acid molecule 13 | Smaller than 0.1 nM * | 12 |
| Control nucleic acid molecule (SEQ ID NO: 14) | 65000 | Not bonded |

**[Table 3]**

| Nucleic acid molecule | Kd (nM) (without dextran) | Kd (nM) (with dextran) |
|---|---|---|
| Binding nucleic acid molecule 3 | Smaller than 0.1 nM * | 0.15 |
| Binding nucleic acid molecule 4 | 0.16 | Smaller than 0.1 nM* |
| Binding nucleic acid molecule 10 | Smaller than 0.1 nM * | - |
| Control Nucleic acid molecule (SEQ ID NO: 14) | 3.3 | - |

As described above, the ability of the SARS-CoV-2 spike glycoprotein-binding nucleic acid molecule of the present invention to bind to the SARS-CoV-2 spike glycoprotein under the condition in a solution containing dextran, which is effective in preventing non-specific binding of nucleic acids, was verified by SPR. In addition, the dynamic parameters could be obtained.

### [Example 3]

The ability of the SARS-CoV-2 spike glycoprotein-binding nucleic acid molecule of the present invention to bind to the SARS-CoV-2 spike glycoprotein was evaluated by Enzyme Linked Aptamer Assay (ELAA).

### (1) Aptamer

Polynucleotides consisting of the base sequences of SEQ ID NOs: 1 and 8 (binding nucleic acid molecule 1 and binding nucleic acid molecule 8) were synthesized in the same manner as in Example 1.

### (2) Sample

The RBD shown in Table 1 of Example 1 was used as a sample.

### (3) Analysis of binding ability by ELAA

The analysis of the binding ability by ELAA was carried out in the same manner as described in Non-Patent Literature 2 (I. Shiratori et al, Biochem Biophys Res Commun 443 (2014) 37-41.). Specifically, the RBD was diluted so as to achieve 1 µg/100µl with 50 mmol/l carbonate buffer (pH 9.6). After the dilution, the RBD was added to the wells of a Maxisorp plate (Thermo Scientific/Nunc, Waltham, MA) at 1 µg/well and solidified at 4°C overnight. After the solidification, the wells were washed once with 200 µl of SB1T buffer (40 mmol/l HEPES (pH7.4), 125 mmol/l NaCl, 1 mmol/l MgCl₂, 5 mmol/l KCl, 0.01% Tween 20). After the washing, the wells were blocked with 200 µl of TBS blocking buffer (Cat No.: 37570, Pierce Biotechnology at room temperature (about 25°C, the same applies hereinafter) for 1 hour. Next, the 5' end of the aptamer was biotinylated in the same manner as in Example 1(3). After the biotinylation, the aptamer was adjusted to 1 µmol/l with the SB1T buffer and thermally denatured at 95°C for 5 minutes. After the denaturation, folding was performed by quenching to 4°C. The aptamer was added to the wells in which the RBD was solidified at 50 µl/well and incubated at 25°C for 1 hour. After the incubation, the wells were washed three times with 200 µl of SB1T buffer. After the washing, 100 µl of streptavidin-horseradish peroxidase (SA-HRP) (Citiva) diluted 1000-fold with the SB1T buffer was added and incubated at 25°C for 30 minutes. After the incubation, the wells were washed three times with the SB1T buffer. After the washing, 100 µl of TMB solution (MOSS) was added, and the mixture was incubated at room temperature for 10 minutes. After the incubation, 0.5 N sulfuric acid was added and the reaction was stopped. Then, the absorbances at 490 nm and 620 nm (background) were measured for the wells using infinite M1000Pro (Tecan). The difference in absorbance between 490 nm and 620 nm (ABS (490nm-620nm)) was calculated. As a control, the difference in absorbance was calculated in the same manner as described above except that the binding nucleic acid molecules 1 and 8 were not added.

The results are shown in FIG. 8. FIG. 8 is a graph showing the ability of the binding nucleic acid molecules 1 and 8 to bind to the RBD. In FIG. 8, the vertical axis indicates the average of the differences in absorbance for three wells of the same sample, the error bars indicate the standard deviations, and the horizontal axis indicates the type of binding nucleic acid molecule and whether or not the RBD was added. In the case of no aptamer, the difference in absorbance was unchanged with or without the addition of RBD. On the other hand, in the binding nucleic acid molecules 1 and 8, the difference in absorbance was increased in the presence of RBD.

From the above, the ability of the binding nucleic acid molecule of the present invention to bind to the SARS-CoV-2 spike glycoprotein could be verified by ELAA.

While the invention has been particularly shown and described with reference to example embodiments and examples thereof, the invention is not limited to these embodiments and examples. It will be understood by those of ordinary skill in the art that various changes in form and details may be made therein without departing from the spirit and scope of the present invention as defined by the claims.

This application is based upon and claims the benefit of priority from Japanese patent application No. 2020-198556, filed on November 30, 2020, the disclosure of which is incorporated herein in its entirety by reference.

### Supplementary Note

The whole or part of the example embodiments and examples disclosed above can be described as, but not limited to, the following supplementary notes.

### (Supplementary Note 1)

A SARS-CoV-2 spike glycoprotein-binding nucleic acid molecule comprising:
any of the following polynucleotides (a), (b), (c) and (d):
(a) a polynucleotide consisting of any of base sequences of SEQ ID NOs: 1 to 7 or a partial sequence of any of the base sequences of SEQ ID NOs: 1 to 7;
(b) a polynucleotide consisting of a base sequence having 80% or more identity to any base sequence of the polynucleotide (a), and binding to a SARS-CoV-2 spike glycoprotein;
(c) a polynucleotide consisting of a base sequence complementary to a polynucleotide that hybridizes to a polynucleotide consisting of any base sequence of the polynucleotide (a) under a stringent condition, and binding to a SARS-CoV-2 spike glycoprotein; and
(d) a polynucleotide consisting of a base sequence obtained by deletion, substitution, insertion, and/or addition of one to several bases in any base sequence of the polynucleotide (a), and binding to a SARS-CoV-2 spike glycoprotein:
   SEQ ID NO: 1: 
GGTATGTCTCCGCCACTGAAATCCGTGCCTAATCTCACCCCACGGAATTCATGGCA
AAGCCGAGGTGTCTTGTATTC;
   SEQ ID NO: 2: 
GGTATGTCTCCGCCACTGAAATCTAATCTCACATTGTAAGCAAAGGAGAATAAGCA
AAGCCGAGGTGTCTTGTATTC;
   SEQ ID NO: 3: 
GGTATGTCTCCGCCACTGAAATCCCTGACCGCTGACCAAATCTCAGTGCAGATGCA
AAGCCGAGGTGTCTTGTATTC;
   SEQ ID NO: 4: 
GGTATGTCTCCGCCACTGAAATCTTGTCCCCTAATAGGTTCCGACTGACAAGTGCA
AAGCCGAGGTGTCTTGTATTC;
   SEQ ID NO: 5: 
GGTTTAGCCCTCGACTCCAAATGTGCACTCTCCCGGTATCCCTAATCTCACCCGATA
CCGTTGATGTGCTGCCAATAC;
   SEQ ID NO: 6: 
GGTTTAGCCCTCGACTCCAAATGACATGAGCCAGGTGCATCTTGAACGTCATAGAT
ACCGTTGATGTGCTGCCAATAC;
   SEQ ID NO: 7:

GGTTTAGCCCTCGACTCCAAATGTGCTGATACTCGGTATCCCTAATCTCACCCGATA
CCGTTGATGTGCTGCCAATAC.

### (Supplementary Note 2)

The SARS-CoV-2 spike glycoprotein-binding nucleic acid molecule according to Supplementary Note 1, wherein
the partial sequence of the base sequence of SEQ ID NO: 1 is the base sequence of SEQ ID NO: 8,
the partial sequence of the base sequence of SEQ ID NO: 2 is the base sequence of SEQ ID NO: 9,
the partial sequence of the base sequence of SEQ ID NO: 3 is the base sequence of SEQ ID NO: 10,
the partial sequence of the base sequence of SEQ ID NO: 5 is the base sequence of SEQ ID NO: 11,
the partial sequence of the base sequence of SEQ ID NO: 6 is the base sequence of SEQ ID NO: 12, or
the partial sequence of the base sequence of SEQ ID NO: 7 is the base sequence of SEQ ID NO: 13:
   SEQ ID NO: 8: CCACTGAAATCCG**T**GCC**T**AA**T**C**T**CACCCCACGGAA**TT**CA**T**GG;
   SEQ ID NO: 9: TCCGCCACTGAAATC**T**AA**T**C**T**CACA**TT**G**T**AAGCAAAGGAGAA**T**AA;
   SEQ ID NO: 10: CCACTGAAATCCC**T**GACCGC**T**GACCAAA**T**C**T**CAG**T**GCAGA**T;**
   SEQ ID NO: 11: TGTGCACTCTCCCGG**T**A**T**CCC**T**AA**T**C**T**CACCCGA**T**ACC;
   SEQ ID NO: 12:
      TGACATGAGCCAGG**T**GCA**T**C**TT**GAACG**T**CA**T**AGA**T**ACCG**TT**GA**T**G**T**GC**T**G;
   SEQ ID NO: 13: GCTGATACTCGG**T**A**T**CCC**T**AATC**T**CACCCGA**T**ACCG.

### (Supplementary Note 3)

The SARS-CoV-2 spike glycoprotein-binding nucleic acid molecule according to Supplementary Note 1 or 2, wherein
the polynucleotide (b) is the following polynucleotide (b1):
(b1) a polynucleotide consisting of a base sequence having 80% or more identity to any base sequence of the polynucleotide (a), comprising any of the base sequences of SEQ ID NOs: 8 to 13, and binding to a SARS-CoV-2 spike glycoprotein.

### (Supplementary Note 4)

The SARS-CoV-2 spike glycoprotein-binding nucleic acid molecule according to Supplementary Note 1 or 2, wherein
the polynucleotide (d) is the following polynucleotide (d1):
(d1) a polynucleotide consisting of a base sequence obtained by deletion, substitution, insertion, and/or addition of one to several bases in any base sequence of the polynucleotide (a), comprising any of the base sequences of SEQ ID NOs: 8 to 13, and binding to a SARS-CoV-2 spike glycoprotein.

### (Supplementary Note 5)

The SARS-CoV-2 spike glycoprotein-binding nucleic acid molecule according to any one of Supplementary Notes 1 to 4, wherein
the binding nucleic acid molecule comprises a modified base being a base modified with a modifying group.

### (Supplementary Note 6)

The SARS-CoV-2 spike glycoprotein-binding nucleic acid molecule according to Supplementary Note 5, wherein
the modified base is a modified thymine base being a thymine base modified with a modifying group.

### (Supplementary Note 7)

The SARS-CoV-2 spike glycoprotein-binding nucleic acid molecule according to Supplementary Note 6, wherein
the modified thymine base is a modified thymine base represented by the following formula (1):

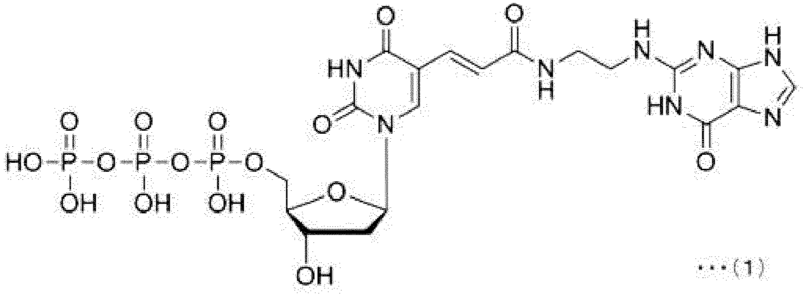

### (Supplementary Note 8)

The SARS-CoV-2 spike glycoprotein-binding nucleic acid molecule according to any one of Supplementary Notes 5 to 7, wherein
underlined thymine bases in the base sequences of SEQ ID NOs: 1 to 13 in the polynucleotide are modified bases.

### (Supplementary Note 9)

The SARS-CoV-2 spike glycoprotein-binding nucleic acid molecule according to any one of Supplementary Notes 1 to 8, wherein
the polynucleotide is a DNA.

### (Supplementary Note 10)

A SARS-CoV-2 detection sensor comprising:
the SARS-CoV-2 spike glycoprotein-binding nucleic acid molecule according to any one of Supplementary Notes 1 to 9.

### (Supplementary Note 11)

A SARS-CoV-2 detection reagent comprising:
the SARS-CoV-2 spike glycoprotein-binding nucleic acid molecule according to any one of Supplementary Notes 1 to 9.

### (Supplementary Note 12)

A method for detecting SARS-CoV-2 comprising:
detecting a SARS-CoV-2 spike glycoprotein in the sample by bringing a sample into contact with a nucleic acid molecule; wherein
the nucleic acid molecule is the SARS-CoV-2 spike glycoprotein-binding nucleic acid molecule according to any one of Supplementary Notes 1 to 9, and
in the detecting, the SARS-CoV-2 spike glycoprotein in the sample is bonded to the nucleic acid molecule to detect the SARS-CoV-2 spike glycoprotein in the sample.

### (Supplementary Note 13)

The method for detecting SARS-CoV-2 according to Supplementary Note 12, wherein
the sample is at least one selected from the group consisting of aerosols, saliva, urine, plasma, and serum.

### (Supplementary Note 14)

A SARS-CoV-2 virus deactivator or neutralizer comprising:
the SARS-CoV-2 spike glycoprotein-binding nucleic acid molecule according to any one of Supplementary Notes 1 to 9.

### Industrial Applicability

As described above, a SARS-CoV-2 spike glycoprotein-binding nucleic acid molecule according to an example aspect of the invention is capable of specifically binding to a SARS-CoV-2 spike glycoprotein, and having high binding ability and slow dissociation. Therefore, according to a SARS-CoV-2 spike glycoprotein-binding nucleic acid molecule according to an example aspect of the invention, for example, the SARS-CoV-2 spike glycoprotein can be detected with excellent accuracy by whether or not the SARS-CoV-2 spike glycoprotein-binding nucleic acid molecule is bonded to a SARS-CoV-2 spike glycoprotein in the sample. Thus, the SARS-CoV-2 spike glycoprotein-binding nucleic acid molecule of the present invention is very useful for the detection of SARS-CoV-2 in the fields of preventive medicine, healthcare, diagnosis of infectious diseases, and the like. The SARS-CoV-2 spike glycoprotein-binding nucleic acid of the present invention can be used, for example, for detecting viruses in biological samples and aerosols, and for inactivating viruses.

## Claims

1. A SARS-CoV-2 spike glycoprotein-binding nucleic acid molecule, comprising:
any of the following polynucleotides (a), (b), (c) and (d):
(a) a polynucleotide consisting of any of base sequences of SEQ ID NOs: 1 to 7 or a partial sequence of any of the base sequences of SEQ ID NOs: 1 to 7;
(b) a polynucleotide consisting of a base sequence having 80% or more identity to any base sequence of the polynucleotide (a), and binding to a SARS-CoV-2 spike glycoprotein;
(c) a polynucleotide consisting of a base sequence complementary to a polynucleotide that hybridizes to a polynucleotide consisting of any base sequence of the polynucleotide (a) under a stringent condition, and binding to a SARS-CoV-2 spike glycoprotein; and
(d) a polynucleotide consisting of a base sequence obtained by deletion, substitution, insertion, and/or addition of one to several bases in any base sequence of the polynucleotide (a), and binding to a SARS-CoV-2 spike glycoprotein:
SEQ ID NO: 1: 
GGTATGTCTCCGCCACTGAAATCCGTGCCTAATCTCACCCCACGGAATTCATGGCA
AAGCCGAGGTGTCTTGTATTC;
SEQ ID NO: 2: 
GGTATGTCTCCGCCACTGAAATCTAATCTCACATTGTAAGCAAAGGAGAATAAGCA
AAGCCGAGGTGTCTTGTATTC;
SEQ ID NO: 3: 
GGTATGTCTCCGCCACTGAAATCCCTGACCGCTGACCAAATCTCAGTGCAGATGCA
AAGCCGAGGTGTCTTGTATTC;
SEQ ID NO: 4: 
GGTATGTCTCCGCCACTGAAATCTTGTCCCCTAATAGGTTCCGACTGACAAGTGCA
AAGCCGAGGTGTCTTGTATTC;
SEQ ID NO: 5: 
GGTTTAGCCCTCGACTCCAAATGTGCACTCTCCCGGTATCCCTAATCTCACCCGATA
CCGTTGATGTGCTGCCAATAC;
SEQ ID NO: 6: 
GGTTTAGCCCTCGACTCCAAATGACATGAGCCAGGTGCATCTTGAACGTCATAGAT
ACCGTTGATGTGCTGCCAATAC;
SEQ ID NO: 7:

GGTTTAGCCCTCGACTCCAAATGTGCTGATACTCGGTATCCCTAATCTCACCCGATA
CCGTTGATGTGCTGCCAATAC.

2. The SARS-CoV-2 spike glycoprotein-binding nucleic acid molecule according to claim 1, wherein
the partial sequence of the base sequence of SEQ ID NO: 1 is the base sequence of SEQ ID NO: 8,
the partial sequence of the base sequence of SEQ ID NO: 2 is the base sequence of SEQ ID NO: 9,
the partial sequence of the base sequence of SEQ ID NO: 3 is the base sequence of SEQ ID NO: 10,
the partial sequence of the base sequence of SEQ ID NO: 5 is the base sequence of SEQ ID NO: 11,
the partial sequence of the base sequence of SEQ ID NO: 6 is the base sequence of SEQ ID NO: 12, or
the partial sequence of the base sequence of SEQ ID NO: 7 is the base sequence of SEQ ID NO: 13:
SEQ ID NO: 8: CCACTGAAATCCG**T**GCC**T**AA**T**C**T**CACCCCACGGAA**TT**CA**T**GG;
SEQ ID NO: 9: TCCGCCACTGAAATC**T**AA**T**C**T**CACA**TT**G**T**AAGCAAAGGAGAA**T**AA;
SEQ ID NO: 10: CCACTGAAATCCC**T**GACCGC**T**GACCAAA**T**C**T**CAG**T**GCAGA**T;**
SEQ ID NO: 11: TGTGCACTCTCCCGG**T**A**T**CCC**T**AA**T**C**T**CACCCGA**T**ACC;
SEQ ID NO: 12:
TGACATGAGCCAGG**T**GCA**T**C**TT**GAACG**T**CA**T**AGA**T**ACCG**TT**GA**T**G**T**GC**T**G;
SEQ ID NO: 13: GCTGATACTCGG**T**A**T**CCC**T**AA**T**C**T**CACCCGA**T**ACCG.

3. The SARS-CoV-2 spike glycoprotein-binding nucleic acid molecule according to claim 1 or 2, wherein
the polynucleotide (b) is the following polynucleotide (b1):
(b1) a polynucleotide consisting of a base sequence having 80% or more identity to any base sequence of the polynucleotide (a), comprising any of the base sequences of SEQ ID NOs: 8 to 13, and binding to a SARS-CoV-2 spike glycoprotein.

4. The SARS-CoV-2 spike glycoprotein-binding nucleic acid molecule according to claim 1 or 2, wherein
the polynucleotide (d) is the following polynucleotide (d1):
(d1) a polynucleotide consisting of a base sequence obtained by deletion, substitution, insertion, and/or addition of one to several bases in any base sequence of the polynucleotide (a), comprising any of the base sequences of SEQ ID NOs: 8 to 13, and binding to a SARS-CoV-2 spike glycoprotein.

5. The SARS-CoV-2 spike glycoprotein-binding nucleic acid molecule according to any one of claims 1 to 4, wherein
the binding nucleic acid molecule comprises a modified base being a base modified with a modifying group.

6. The SARS-CoV-2 spike glycoprotein-binding nucleic acid molecule according to claim 5, wherein
the modified base is a modified thymine base being a thymine base modified with a modifying group.

7. The SARS-CoV-2 spike glycoprotein-binding nucleic acid molecule according to claim 6, wherein
the modified thymine base is a modified thymine base represented by the following formula (1):

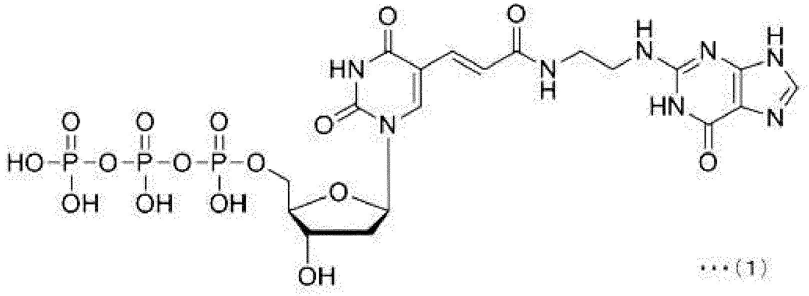

8. The SARS-CoV-2 spike glycoprotein-binding nucleic acid molecule according to any one of claims 5 to 7, wherein
underlined thymine bases in the base sequences of SEQ ID NOs: 1 to 13 in the polynucleotide are modified bases.

9. The SARS-CoV-2 spike glycoprotein-binding nucleic acid molecule according to any one of claims 1 to 8, wherein
the polynucleotide is a DNA.

10. A SARS-CoV-2 detection sensor, comprising:
the SARS-CoV-2 spike glycoprotein-binding nucleic acid molecule according to any one of claims 1 to 9.

11. A SARS-CoV-2 detection reagent, comprising:
the SARS-CoV-2 spike glycoprotein-binding nucleic acid molecule according to any one of claims 1 to 9.

12. A method for detecting SARS-CoV-2, comprising:
detecting a SARS-CoV-2 spike glycoprotein in the sample by bringing a sample into contact with a nucleic acid molecule; wherein
the nucleic acid molecule is the SARS-CoV-2 spike glycoprotein-binding nucleic acid molecule according to any one of claims 1 to 9, and
in the detecting, the SARS-CoV-2 spike glycoprotein in the sample is bonded to the nucleic acid molecule to detect the SARS-CoV-2 spike glycoprotein in the sample.

13. The method for detecting SARS-CoV-2 according to claim 12, wherein
the sample is at least one selected from the group consisting of aerosols, saliva, urine, plasma, and serum.

14. A SARS-CoV-2 virus deactivator, comprising:
the SARS-CoV-2 spike glycoprotein-binding nucleic acid molecule according to any one of claims 1 to 9.
